# EUROPÄISCHE PATENTSCHRIFT

(11) **EP 1 492 545 B1**
(45) Veröffentlichungstag und Bekanntmachung des Hinweises auf die Patenterteilung: **15.09.2010**
(21) Anmeldenummer: 03720269.4
(22) Anmeldetag: 04.04.2003
(51) Int. Cl.: A61K 31/69, A61K 31/11, A61K 38/06, A61K 31/16

(54) **MITTEL ZUR BEHANDLUNG VON FLAVIVIRIDAE-INFEKTIONEN**
AGENTS FOR TREATING I FLAVIVIRIDAE /I INFECTIONS
AGENT DE TRAITEMENT D'INFECTIONS PAR FLAVIVIRIDAE

(30) Priorität: 05.04.2002 DE 10216227
(43) Veröffentlichungstag der Anmeldung: 05.01.2005
(73) Patentinhaber: ViroLogik GmbH, 91052 Erlangen (DE)
(72) Erfinder: SCHUBERT, Ulrich, 07407 Uhlstädt (DE); WILL, Hans, 22549 Hamburg (DE); SIRMA, Hüseyin, 23553 Elmshorn (DE)
(74) Vertreter: Wehlan, Helmut
(86) Internationale Anmeldenummer: PCT/DE2003/001213
(87) Internationale Veröffentlichungsnummer: WO 2003/084551

(56) Entgegenhaltungen:
- WO-A-02/30455
- WO-A-99/22729
- US-B1- 6 297 217

## Beschreibung

Die Erfindung betrifft Mittel (Proteasom-Inhibitoren) zur Vorbeugung, Behandlung, Therapie und Hemmung der von verschiedenen Mitgliedern der Virusfamilie *Flaviviridae* (Genera: *Flavivirus, Pestivirus*) verursachten Krankheiten beim Menschen und bei Tieren. Darüber hinaus betrifft dies die von Pestiviren wie BVDV (Virus der bovinen viralen Diarrhoe) und CSFV (Virus der klassischen Schweinepest) verursachten Krankheiten bei Tieren und die von Flaviviren wie zum Beispiel West-Nil-, Dengue- und Frühsommer-Meningoenzephalitis (FSME) Viren verursachten Krankheiten bei Menschen und Tieren.

Die Anwendung der erfindungsgemäßen Mittel in pharmazeutischen Zubereitungen führt vorwiegend oder ausschließlich zur Freisetzung von nicht infektiösen Viren aus infizierten Zellen. Die gleichen Mittel können die Ausbreitung einer akuten Infektion in Zellkulturen wie auch im infizierten Organismus begrenzen, da alle Nachkommenviren, produziert unter Behandlung mit diesen Mitteln, nicht oder so gut wie nicht infektiös sind. Bei den Mitteln handelt es sich um verschiedene Substanzklassen, welchen gemeinsam ist, dass sie das 26S Proteasom in Zellen inhibieren. Am Beispiel von Vertretern der Genera *Flavivirus* und *Pestivirus* wird gezeigt, dass diese Mittel, die Proteasom-Inhibitoren, die Freisetzung von infektiösen Viren aus infizierten Wirtszellen drastisch reduzieren. Das Pestivirus BVDV dient dabei aufgrund einer ausgeprägten Homologie sowohl der Struktur des Viruspartikels als auch der Genomorganisation als Surrogat-Modell für das HCV, für das selbst kein infektiöses Zellsystem zur Verfügung steht. Anwendungsbeispiele betreffen die Behandlung von BVDV-Infektionen beim Rind und CSFV-Infektionen beim Schwein. Darüber können diese Mittel auch bei der Vorbeugung und Behandlung von Infektionskrankheiten Anwendung finden, die von Flaviviren wie West-Nil-Virus, Gelbfieber-Virus, Dengue-Virus und Frühsommer-Meningoenzephalitis (FSME) Virus verursacht werden. Dem bisherigen Erkenntnisstand zufolge ist davon auszugehen, dass mit Proteasom-Inhibitoren die Virämie sowohl bei einer Neuinfektion als auch bei chronischen Infektionen unterdrückt und der Erfolg einer Viruseliminierung durch das eigene Immunsystem und/oder durch bekannte Mittel mit ähnlicher oder anderer Wirkung erhöht werden kann.

### Charakteristik des bekannten Standes

### 0. Einleitung

Eines der größten Weltgesundheitsprobleme (ca. 3% der Weltbevölkerung sind betroffen) ist die Infektion mit Hepatitis-C-Virus (HCV). Wichtigstes Merkmal von HCV-Infektionen ist ein oft lebenslang andauernder chronischer Virus-Trägerstatus der betroffenen Personen: Mehr als 70% der HCV-Infizierten entwickeln eine chronische Hepatitis, die zu chronisch aktiver Hepatitis verschiedener Schweregrade bis hin zu Leberzirrhose und Fibrose (10-20% aller Fälle) führen kann. Unabhängig davon entstehen bei 1 bis 5% der chronisch Infizierten primäre hepatozelluläre Karzinome (HCC; für Reviews siehe Lindenbach and Rice, 2001 sowie Major *et al.,* 2001). Darüber hinaus werden HCV-Infektionen ursächlich noch mit mehr als 30 weiteren Erkrankungen beim Menschen in Verbindung gebracht, darunter mit bedeutenden Autoimmunerkrankungen wie der Typ II Kryoglobulinämie (mixed type II cryoglobulinemia, MC; Agnello, 2000). HCV-Infizierte werden in der Regel mit einer Kombination von Interferon-alpha und Ribavirin behandelt. Diese Methode ist aber mit starken Nebenwirkungen behaftet und führt nur in etwa der Hälfte aller Fälle zur Eliminierung des Virus aus dem Körper. Diese unbefriedigende Situation liegt zum einen an der generell breiten Vielfalt der HCV-Varianten: Zur Zeit werden sechs Genotypen unterschieden; diese lassen sich wiederum in eine Reihe von Subtypen und Stämmen diversifizieren (für Review siehe Bukh *et al.,* 1995). Zum anderen entwickeln sich in den chronisch infizierten Individuen eine Vielzahl neuer genetischer Varianten. Die genetische Variabilität von HCV ist wahrscheinlich auch die Ursache für die ineffiziente Immunantwort, die bei der Mehrheit der HCV-Infizierten beobachtet werden kann. Aus diesem Grund konnten bisher weder prophylaktische noch therapeutische Impfmethoden entwickelt werden. Auch die passive Gabe von HCV-spezifischen neutralisierenden Antikörpern und/oder Medikamenten bei Lebertransplantierten verhindert oft nicht die Neuinfektion der transplantierten Leber. Immunsuppression von Patienten mit abklingenden Hepatitiden oder nach einer Lebertransplantation kann zur Reaktivierung von latent vorhandenen Viren führen. Um die Probleme der bisher verfügbaren antiviralen und therapeutischen Mittel für die Hepatitis C zu umgehen, sind neue Therapieansätze notwendig. Vor dem Hintergrund der Virusvariabilität sind dabei vor allem solche Methoden von großer Bedeutung, die konservierte zelluläre Faktoren beeinflussen, die für die Vermehrung dieser Viren in der Wirtszelle essentiell sind. Solche Mittel werden in dieser Erfindung beschrieben. Da bisher weder effizient infizierbare Zellen in Kultur noch ein praktikables Tiermodell für HCV zur Verfügung stehen, wurden hierzu Studien mit dem HCV-verwandten Pestivirus BVDV durchgeführt. Die Ähnlichkeit des Lebenszyklus von BVDV und HCV wurde in einer Reihe vorangehender Arbeiten demonstriert (für Review siehe Lindenbach and Rice, 2001). Darüber hinaus wurden Experimente mit einem anderen, phylogenetisch weiter entfernten Vertreter der *Flaviviridae* Familie, dem West-Nil Flavivirus, durchgeführt. Die Erfindung betrifft die überraschende Erkenntnis, dass Inhibitoren des zellulären 26S Proteasoms die Produktion von infektiösen Viren sowohl aus BVDV als auch aus West-Nil-Virus-infizierten Zellen verhindern. Der homologe Effekt von Proteasom-Inhibitoren auf die Vermehrung von Pestiviren und Flaviviren impliziert einen ähnlichen Wirkmechanismus dieser Mittel bei allen Vertretern der *Flaviviridae* Familie; dieser beruht wahrscheinlich ursächlich auf der homologen Struktur der Virionen der *Flaviviridae.* Neben der antiviralen Wirkung wurde festgestellt, dass der zytotoxische Effekt von Proteasom-Inhibitoren bei Hepatomazellen (Leberkrebszellen), die sich schnell teilen, signifikant stärker ausgeprägt ist als bei langsam teilenden primären Hepatozyten. Aufgrund dieser Eigenschaften sind Proteasom-Inhibitoren besonders für die Therapie von HCV-verursachten Lebererkrankungen und Leberkarzinomen geeignet. Die Wirkung von Proteasomen-Inhibitoren auf Pestiviren legt nahe, diese Mittel bei der Bekämpfung der verbreiteten Tierpathogene BVDV (Ursache von boviner Virus-Diarrhoe und boviner mucosal disease, MD) und CSFV (Ursache der Schweinepest) einzusetzen (für Review siehe Thiel, 1996). Die Wirkung von Proteasom-Inhibitoren auf die Vermehrung von Flaviviren eröffnet Anwendungsmöglichkeiten bei zahlreichen durch Flaviviren verursachten Krankheiten beim Menschen und bei Tieren, darunter Fieber, Enzephalitiden und Hämorrhagien. Bedeutungsvoll ist in diesem Kontext vor allem das Dengue-Fieber-Virus, gegen das es bisher nur unzureichende Vakzine und Behandlungsformen gibt (für Review, siehe Burke and Monath, 2001).

### 1. Funktion des Ubiquitin/Proteasom-Pathway

Proteasomen stellen die hauptsächliche proteolytische Komponente im Zellkern und Zytosol aller eukaryotischen Zellen dar. Es sind multikatalytische Enzymkomplexe, die ca. 1% der Gesamt-Zellproteine ausmachen. Proteasomen üben eine vitale Rolle in vielfältigen Funktionen des Zellmetabolismus aus. Die hauptsächliche Funktion ist die Proteolyse von missgefalteten, nicht-funktionellen Proteinen und der schnelle Abbau regulatorischer Proteine. Eine weitere Funktion hat der proteasomale Abbau von zellulären oder viralen Proteinen für die T-Zell-vermittelte Immunantwort durch die Generierung von Peptidliganden für Major Histocompatibilitäts Klasse-I-Moleküle (für Review siehe Rock und Goldberg, 1999). Proteasom-Targets werden in der Regel durch die Anheftung von oligomeren Formen von Ubiquitin (Ub) für den Abbau markiert. Ub ist ein hochkonserviertes, 76 Aminosäuren langes Protein, das kovalent an Targetproteine gekoppelt wird. Die Ubiquitinylierung selbst ist reversibel, und Ub-Moleküle können durch eine Vielzahl von Ub-Hydrolasen von dem Targetmolekül wieder entfernt werden. Die Verbindung zwischen der Ubiquitinylierung von Targetproteinen und der proteasomalen Proteolyse wird allgemein als Ubiquitin/Proteasom-System (UPS) bezeichnet (für Review siehe Rock und Goldberg, 1999; Hershko und Ciechanover, 1998).

Das 26S Proteasom ist ein 2.5 Mda großer Multienzym-Komplex, welcher aus ca. 31 Untereinheiten besteht. Die proteolytische Aktivität des Proteasom-Komplexes wird durch eine zylinderförmige, 700 kDa große und aus vier übereinander liegenden Ringen bestehende Core-Struktur, dem 20S Proteasom, realisiert. Das 20S Proteasom bildet einen aus 14 nicht identischen Proteinen bestehenden komplizierten Multienzymkomplex, der in zwei α-und zwei β-Ringen in einer αββα-Reihenfolge angeordnet ist. Die Substratspezifität des 20S Proteasom umfasst drei wesentliche Aktivitäten: Trypsin-, Chymotrypsin- und Postglutamyl-Peptid hydrolysierende- (PGPH) oder auch Kaspase-ähnliche Aktivitäten, welche in den β-Untereinheiten Z, Y und Z lokalisiert sind. Das 20S Proteasom degradiert in vitro denaturierte Proteine unabhängig von deren Poly-Ubiquitinylierung. Dagegen werden in vivo enzymatische Aktivitäten des 20S Proteasoms durch Anlagerung der 19S regulatorischen Untereinheiten reguliert, welche zusammen das aktive 26S Proteasom Partikel bilden. Die 19S regulatorischen Untereinheiten sind bei der Erkennung von poly-ubiquitinylierten Proteinen sowie bei der Entfaltung von Targetproteinen beteiligt. Die Aktivität des 26S Proteasom ist ATPabhängig und degradiert fast ausschließlich nur poly-ubiquitinylierte Proteine (für Review siehe Hershko und Ciechanover, 1998).

### 1.1. Bedeutung des UPS in der Pathogenese klinisch relevanter Krankheiten

Die zentrale Rolle des UPS in der Zelle erklärt die Bedeutung dieses Systems für zahlreiche pathologische Erscheinungen (für Review siehe Ciechanover et al., 2000). Beispielsweise ist der Level des Tumorsuppressor-Proteins p53 in besonders aggressiven Formen von zervikalen Karzinomen extrem niedrig, die durch bestimmte Isolate des Humanen Papilloma-Virus (HPV) ausgelöst werden. Das HPV onco-Protein E6 induziert den Abbau des Suppressorproteins p53 über das UPS.

Bei der Entstehung von colorektalem Cancer spielt β-Catenin, ein über das UPS regulierter zellulärer Faktor, eine wichtige Rolle in der Signal-Transduktion und Differenzierung von colorektalem Epithelium. Außerdem besteht eine Korrelation zwischen dem Level an p27, einem G1-Cyclin CDK-Inhibitor und dem Entstehen von colorektalen Cancer und Brustkrebs. Der Abbau von p27 durch das UPS ist entscheidend für den Übergang von G1- zur S-Phase während der Zellteilung.

Eine Rolle des UPS bei Erbkrankheiten ist bekannt, so für den Pathomechanismus von Cystischer Fibrosis, dem Angelmans-Syndrom sowie dem Liddle-Syndrom. Auch bei neurodegenerativen Erkrankungen spielt das UPS eine Rolle: Die Akkumulation von Ubiquitin-Konjugaten wurde in pathologischen Läsionen bei Alzheimer und Parkinson berichtet. Bei Huntington akkumulieren die Proteine Huntingtin und Ataxin in Proteasom-aktiven nukleären Strukturen im Zellkern. Eine zentrale Funktion übt das UPS bei Erkrankungen des Immunsystems aus. Zum einen ist der 26S Proteasom-Komplex die hauptsächliche Protease in der MHC-I-Antigenprozessierung, und zum anderen kann die Aktivität des Proteasoms selbst sowohl durch γ-Interferon induzierbare katalytische β-Untereinheiten als auch durch die regulatorische Untereinheit PA28 manipuliert werden. Viele entzündliche und immunologische Krankheiten stehen im Zusammenhang mit dem Transkriptionsfaktor NF-κB, welcher verschiedene Gen-Funktionen in der Immunantwort reguliert. Die Aktivierung von NF-κB, die durch Ubiquitinylierung und spezifische Spaltung eines Vorläuferproteins durch das Proteasom gesteuert wird, führt zur erhöhten Expression von verschiedenen Zytokinen, Adhäsionsmolekülen, entzündlichen und Stress-Response-Proteinen sowie Immunrezeptoren.

Diese Zusammenhänge erklären das Interesse an einer pharmakologischen Anwendung von Substanzen, die das UPS regulieren.

### 1.2. Proteasom-Inhibitoren

Verschiedene Substanzklassen sind als Proteasom-Inhibitoren bekannt. Es sind zum einen chemisch modifizierte Peptidaldehyde wie der Tripeptidaldehyd N-carbobenzoxyl-L-leucinyl-L-leucinyl-L-leucinal (zLLL; auch als MG 132 bezeichnet) sowie das wirksamere Borsäure-Derivat MG232. zLLL und davon abgeleitete Derivate. Sie blockieren das Proteasom reversible durch Ausbildung einer transienten Hemiacetal-Struktur mit der katalytisch aktiven Threonin-Hydroxyl-Seitenkette in Position 1 der β-Untereinheit des 26S Proteasoms. Ähnlich zu zLLL wurde eine weitere Klasse von modifizierten Peptiden, die Peptid-Vinyl-Sulfone, als Proteasom-Inhibitoren beschrieben (für Review siehe Elliott und Ross, 2001).

Natürlich vorkommende Substanzen sind Lactacystin (LC) (Fenteany et al., 1995), das aus Streptomyceten, sowie Epoxomycin, das aus Aktinomyzeten gewonnen wird (Meng et al., 1999a,b). LC ist ein hoch spezifischer, irreversibel wirkender Proteasom-Inhibitor, welcher hauptsächlich die Chymotrypsin und die Trypsin-ähnlichen Aktivitäten des 26S Proteasom-Partikels blockiert (Fenteany et al., 1995). LC hat keine Peptid-Grundstruktur, sondern besteht aus einem γ-Lactam-Ring, einem Cystein und einer Hydroxy-butyl-Gruppe. LC selbst inhibiert nicht das Proteasom. Vielmehr wird in wässriger Lösung der N-Acetyl-Cystein-Rest hydrolysiert. Das Resultat ist die Bildung eines Clastolactacystein β-Lactons, das in der Lage ist, Zellmembranen zu penetrieren. Nach Zellaufnahme kommt es zum nukleophilen Angriff des β-Lacton-Rings und anschließender Transesterifizierung der Threonin1-Hydroxyl-Gruppe der β-Untereinheit (Fenteany et al., 1995). Hinsichtlich Spezifität und Wirksamkeit ist Epoxomycin der bislang wirksamste aller bekannten natürlichen Proteasom-Inhibitoren (Meng et al., 1999;a, b):

Eine weitere und sehr potente Klasse an synthetischen Proteasom-Inhibitoren sind Borsäure-Peptid-Derivate, insbesondere die Verbindung Pyranozyl-Phenyl-Leuzinyl-Borsäure mit dem Namen "PS-341". PS-341 ist unter physiologischen Bedingungen sehr stabil und nach intravenöser Applikation bioverfügbar (Adams und Stein, 1996; Adams et al., 1999, US 1448.012TW01). Die besondere Wirksamkeit von PS-341 als Proteasom-Inhibitor wird vermutlich durch die sehr stabile Bindung zwischen Borsäure- und Hydroxyl-Gruppe der katalytisch aktiven Seitenkette von Thr1 in der aktiven β-Untereinheit des 20S-Proteasoms (Ki = 0.6 nM) realisiert (Adams und Stein, 1996). Außer dem Proteasom ist bislang keine zelluläre Protease bekannt, welche durch PS-341 beeinflusst wird. Verschiedene Borsäure-Peptid-Derivate wurde auf Wirkung als Proteasom-Inhibitor bereits getestet (Adams et al., 1998). Dabei wurde festgestellt, dass Leucin, bevorzugt in P1-Position, sowie relativ große hydrophobe Seitenketten (zum Beispiel Naphthylalanin) in P2 und P3 die Wirksamkeit und den Ki-Wert (inhibitorische Konstante) des Inhibitors verbessern (Adams et al., 1998).

### 1.2.1. Klinische Applikation von Proteasom-Inhibitoren

Die Hemmung der Proteasom-Aktivität als hauptsächliche zelluläre Protease kann zu Veränderungen in der Regulation des Zellzyklus, der Transkription, der gesamten zellulären Proteolyse, sowie der MHC-I Antigenprozessierung führen (für Review siehe Ciechanover et al., 2000). Demzufolge ist eine dauerhafte Inhibierung aller enzymatischen Aktivitäten des Proteasoms mit dem Leben einer Zelle und somit des Gesamtorganismus nicht vereinbar. Bestimmte, reversibel wirkende Proteasom-Inhibitoren können jedoch selektiv einzelne proteolytische Aktivitäten des 26S Proteasoms inhibieren, ohne dabei andere zellulären Proteasen zu beeinflussen. Die Zytotoxizität solcher Inhibitoren ist daher wesentlich geringer im Vergleich zu relativ unspezifisch wirkenden Peptidaldehyden wie zLLL. Erste klinische Studien mit Proteasom-Inhibitoren (Adams et al., 1999) verdeutlichen, dass diese Substanzklasse ein enormes Potential als Pharmaka mit einer vielfältiger Anwendungsbasis hat (für Review siehe Elliot und Ross, 2001).

Die Bedeutung von Proteasom-Inhibitoren als neues therapeutisches Prinzip hat in den vergangenen Jahren zunehmende Aufmerksamkeit erfahren, insbesondere bei der Behandlung von Krebs und entzündlichen Erkrankungen (für Review siehe Elliot und Ross, 2001). Bislang ist der Einsatz für die breite klinische Anwendung am Menschen noch nicht zugelassen, die pharmazeutische Industrie arbeitet aber intensiv an der Entwicklung von neuen Medikamenten auf der Basis von in vivo-verträglichen Proteasom-Inhibitoren. Die Firma "Millennium *Inc.*" (Cambridge, MA, USA) entwickelte Proteasom-Inhibitoren für entzündungshemmende, immunmodulatorische und antineoplastische Therapien, insbesondere Borsäure-Derivate von Di-Peptiden, und dabei insbesondere die Verbindung PS-341 (Adams et al., 1999). Die orale Applikation von PS-341 hat im Ratten-Modell eine entzündungshemmende Wirkung in Streptokokken-induzierter Polyarthritis und Leber-Entzündung (Palombella et al., 1998). Im Maus-Modell zeigt PS-341 anti-neoplastische Wirkung bei Lungenkarzinomen und hat außerdem additive Wirkung in Verbindung mit Zytostatika (Teicher et al., 1999). In vitro-Versuche demonstrierten eine sehr gute Wirksamkeit gegenüber soliden humanen Ovarien- und Prostata-Tumorzellen (Frankel et al., 2000). PS-341 ist der bislang einzige klinisch erprobte Proteasom-Inhibitor. Phase I Studien an PS-341 demonstrierten eine gute BioVerfügbarkeit und pharmakokinetisches Verhalten (Lightcap et al., 2000). Phase I- und Phase II-klinische Studien in Patienten mit unterschiedlichen Krebserkrankungen, wie zum Beispiel hämatologischen Malignancies als soliden Tumoren, wurden bereits abgeschlossen. Neben überraschenden therapeutischen Effekten in verschiedenen Tumor-Patienten ist bemerkenswert, dass keine Dosis-limitierende Toxizität bei der Behandlung mit PS-341 beobachtet werden konnte. Die Informationen dazu wurden in Pressemitteilungen von Millennium *Inc*. vorgestellt. (publiziert unter
http:biz.yahoo.com/prnews/010301/neth003.html;
http:biz.yahoo.com/prnews/010301/neth003.html;
http://www.mlnm.com.releases.pr052300_1.shtml;
http://www.cancernet.nci.nij.gov/;
http://www3.manderson.org/leukemia/insight/letter52.html.

Eine weitere klinische Anwendung von Proteasom-Inhibitoren, insbesondere solche, die von der Firma Millennium *Inc*. entwickelt wurden, deutet sich bei entzündlichen und Auto-Immunerkrankungen an. Diese werden allgemein ausgelöst durch eine Kaskade der Zytokin- und Chemokin-Produktion sowie der Expression von bestimmten Zelladhäsionsmolekülen. Eine zentrale Stellung nimmt hierbei der Transkriptionsfaktor NF-κB ein. Dieser ist notwendig für die Expression einer Reihe von pro-inflammatorischen Faktoren. NF-κB, als ein Vertreter der Rel-Proteine besteht aus einem Heterodimer von p50 und p65 (RelA) Untereinheiten (für Review siehe Baldwin, 1996). In ruhenden Zellen garantiert die Bindung des inhibitorischen Faktors IκB an NF-κB über Maskierung des nukleäres Lokalisationssignal des p50/p65 Heterodimers die Lokalisation von NF-κB im Zytosol und zwar in einer latenten, inaktiven Form. Signale der Zellaktivierung, zum Beispiel Zytokine oder virale Infektionen lösen die Phosphorylierung und poly-Ubiquitinylierung von IκB und damit die Aktivierung von NF-κB aus. Nach Aktivierung translokiert NF-κB in den Zellkern und stimuliert die Transkription von verschiedenen Genen, vor allem von Zytokinen, Chemokinen und Zelladhäsionsmolekülen. Diese Faktoren sind in der Summe involviert in der Regulation von immunologischen und inflammatorischen Prozessen. Durch Proteasom-Inhibitoren kann der Abbau von IκB und somit die Aktivierung von NF-κB blockiert werden (Palombella et al., 1994).

In einem Maus-Modell konnte gezeigt werden, dass der Proteasom-Inhibitor PS-519 (ein β-Lacton-Derivat) eine starke anti-inflammatorische Wirkung ausübt. In niedrigen Dosen ist PS-519 auch wirksam in Kombination mit Steroiden. PS-519 wurde daher als neues Medikament für die Asthma-Behandlung vorgeschlagen (Elliot et al., 1999). Eine weitere Anwendung für PS-519 ergibt sich im Infarkt-Modell: Die inflammatorische Reaktion nach cerebralen Verletzungen wurde durch PS-519 dramatisch reduziert. Danach scheint PS-519 ebenfalls ein interessantes Pharmakon für die Behandlung von Gehirnschlag zu sein (Phillips et al., 2000).

Da Proteasom-Inhibitoren einen essentiellen Pathway im Zellmetabolismus treffen, ist ein strenges Dosis-Regime notwendig, um toxische Neben-Effekte zu unterdrücken. Im Rahmen der Entwicklung von in vivo verträglichen Proteasom-Inhibitoren wurden verschiedene Peptid-Borsäure-Derivate getestet, welche sowohl in der Zellkultur als auch im Tiermodell anti-Tumor-Wirkung zeigten (Adams et al., 1996; 1998; 1999). In vitro besitzt PS-341 eine selektive zytotoxische Aktivität gegen ein breites Spektrum an humanen Tumorzelllinien (Adams et al., 1999). Diese Aktivität ist mit der Akkumulation von p21 und Zellzyklus-Arrest in der G2-M-Phase mit nachfolgender Apoptose verbunden (Adams et al., 1999). Direkte Injektion von PS-341 bewirkte im Maus-Modell das Absterben von 70% der untersuchten Tumoren. Nach intravenöser Verabreichung von PS-341 verteilte sich die Substanz in allen Organen und Geweben und hatte anti-neoplastische Aktivität in human-Xenograft-Modellen (Adams et al., 1999). Toxikologische Studien zu PS-341 in Primaten erbrachten Dosis-abhängige Nebenwirkungen vor allem im gastrointestinalen Bereich, wo PS-341 nach intravenöser Applikation die höchste Verteilung zeigte (Adams et al., 1999). Ein weiterer Nachteil von PS-341 und verwandten Inhibitoren ist, dass sie nicht die Blut-Hirnschranke überwinden und somit nicht im Zentralnervensystem wirksam werden können (Adams et al., 1999).

Der Einsatz von Proteasom-Inhibitoren mit dem Ziel, virale Infektionen zu blockieren, wurde bereits beschrieben. Insbesondere wurde von Schubert et al. (2000 a, b) gezeigt, dass Proteasom-Inhibitoren die Assemblierung, Freisetzung und proteolytische Reifung von HIV-1 und HIV-2 blockieren. Dieser Effekt beruht auf einer spezifischen Blockade der proteolytischen Prozessierung der Gag-Polyproteine durch die HIV-Protease, ohne dass Proteasom-Inhibitoren die enzymatische Aktivität der viralen Protease selbst beeinflussen. Der Mechanismus, mit welchem Proteasomen die Assemblierung verschiedener Viren regulieren, ist bislang unverstanden. Weitere Zusammenhänge mit dem UPS wurden für Budding von Rous Sarcoma Virus, RSV (Patnaik et al., 2000); Simian Immunodeficiency Virus, SIV (Strack et al., 2000), und Ebola Virus (Harty et al., 2000) berichtet. Im letzteren Fall (Harty et al., 2000) wurde gezeigt, dass eine zelluläre Ubiquitinligase mit Ebola-Matrixprotein in Wechselwirkung tritt, eine direkte Bedeutung der Proteasom-Aktivität für die Replikation von Ebola oder verwandten Viren sowie die Inhibierung der Assemblierung von Ebola-Viren wurde bislang nicht gezeigt.

Ein wesentlicher Teil der Erfindung besteht also darin, dass die überraschenderweise festgestellte anti-virale Wirkung von Proteasom-Inhibitoren auf Mitglieder der *Flaviviridae* Familie, speziell auf mit dem HCV nächst-verwandte Viren wie Pestiviren, bislang nicht beschrieben wurde. Insbesondere wurden keinerlei anti-virale Effekte durch Proteasom-Inhibitoren beschrieben, die den Eintritts-/Internalisierungsprozess oder das Uncoating (Freisetzen des Virus-core) des *Flaviviridae*-Viruspartikels betreffen. Ebensowenig wurden anti-virale Effekte durch Proteasom-Inhibitoren beschrieben, die die Assemblierung, Maturierung oder die Sekretion von Nachkommenviren von *Flaviviridae* betreffen. Es wurde nicht beschrieben, dass Proteasom-Inhibitoren die Freisetzung von infektiösen *Flaviviridae-*Virionen der Virus-Produzent-Zellen blockieren. Weiterhin wurde nicht berichtet, dass Proteasom-Inhibitoren im Vergleich zu primären Hepatozyten bevorzugt Hepatomazellen abtöten und dadurch für die Therapie von Leberkarzinomen geeignet sind. Die erfindungsgemäß dargestellten Wirkungen von Proteasom-Inhibitoren auf frühe und/oder späte Prozesse des *Flavivi*ridae-Lebenszyklus, stellen somit völlig neuartige Prinzipien der anti-viralen Behandlung von Infektionen dieser Viren, speziell von HCV-Infektionen, dar.

### 1.3. Verbindung zwischen dem UPS und dem Lebenszyklus von Flaviviridae, speziell HCV

In keiner bekannten Studie wurde bisher analysiert, welchen Einfluss Proteasom-Inhibitoren auf die Freisetzung und Infektiosität von Mitgliedern der *Flaviviridae-Familie* haben, wie dies in der vorliegenden Erfindungsbeschreibung beobachtet und dargestellt wird. Publiziert wurde bisher, dass die Expression von HCV-Proteinen die Aktivität des UPS nicht beeinflusst (Moradpour *et al.,* 2001). Darüber hinaus ist bekannt, dass eine Fraktion des HCV-Core-Proteins über das UPS abgebaut wird (Suzuki *et al.,* 2001). Es tritt auch eine mono-ubiquitinierte Form des Core-Proteins auf, die jedoch stabil ist und nicht abgebaut wird. Bisher wurde nicht getestet, ob Proteasom-Inhibitoren einen Einfluss auf die Proliferation oder den Transformationstatus von hepatozelluläre Karzinomen (HCC) haben. Bekannt ist lediglich, dass die P28 Proteasom-Untereinheit in den meisten HCCs überexprimiert wird (Higashitsuji *et al.,* 2000).

### 2. Biologie der Flaviviridae

Die Familie *Flaviviridae* umfasst die Genera *Flavivirus, Pestivirus* und *Hepacivirus* (Hepatitis-C-Virus, HCV). Neben diesen drei Genera wurde kürzlich eine Gruppe weiterer *Flaviviridae-*ähnlicher Viren, die GB-Viren, charakterisiert, die bisher nicht klassifiziert sind. Die phylogenetische Verwandtschaft ist zwischen Pestiviren und HCV erheblich ausgeprägter als zwischen Flavi- und Pesti- bzw. HCV und Flaviviren. Dementsprechend sind die Morphologie des Virions, die Genomorganisation und die bekannten biochemischen Mechanismen der Genexpression und Genomreplikation zwischen Pestiviren und HCV besonders konserviert. Wie bereits ausgeführt, handelt es sich bei vielen Mitgliedern der *Flaviviridae* um bedeutende Human- und Tierpathogene (für Review siehe Lindenbach and Rice, 2001).

### 2.1. Aufbau des Virions; Mechanismus des Virus-Eintritts in die Wirtszelle

Das Virusgenom der *Flaviviridae* ist eine einzelsträngige, nicht segmentierte RNA positiver Orientierung mit einer Länge von 10 (HCV), 11 (Flaviviren) bzw. 13 Kilobasen (Pestiviren). Im Virion ist das RNA-Genom mit Kapsid(C, oder "Core")- Proteinen komplexiert. Dieser Nukleoprotein-Komplex wird von einer Lipid-Doppelmembran umhüllt, in die zwei oder drei Spezies von Hüll(E, oder "Envelope")-Proteinen eingelagert sind. Es wird vermutet, dass Bindung des Viruspartikels an die Zelle und Eintritt ("Entry") über eine Rezeptor-mediierte Endozytose erfolgt. Die Virus-Hüllproteine assoziieren dabei mit einem zellulären Rezeptor: Verschiedene experimentelle Daten weisen bei HCV auf eine Interaktion des Envelope-Proteins E2 mit CD81 und/oder dem low density lipoprotein(LDL)-Rezeptor (für Review siehe Lindenbach and Rice, 2001). Bei BVDV wurde eine Interaktion mit CD 46 und dem LDL-Rezeptor festgestellt (Müller et al. 2000; Agnello et al., 2000). Durch Fusion der Virushülle mit der Membran, katalysiert durch niedrigen pH-Wert, kommt es zur Freisetzung des Nukleokapsids in das Zytoplasma. Ein unbekannter Mechanismus führt zur Dissoziation der viralen Kapsidproteine und zur Freisetzung (Uncoating - Freisetzung, Entmantelung des Virus-core) des Genoms (für Review siehe Lindenbach and Rice, 2001).

### 2.2. Genexpression der Flaviviridae

Die virale RNA umfasst ein einziges translationales Leseraster ("open reading frame"; ORF), das am 5'- und am 3'-Ende von nicht-translatierten Regionen (UTRs) umrahmt wird. Die Translation führt zur Synthese eines instabilen Polyproteins, das ko- und posttranslational in virale Polypeptide gespalten wird.

Entsprechend der Ähnlichkeiten der Genomstruktur sind bei den Mitgliedern der *Flaviviridae* auch die Vorgänge, die zur Prozessierung des Polyproteins führen, weitgehend konserviert. Dies gilt insbesondere für Pestiviren und HCV. Der aminoterminale Bereich des Polyproteins enthält die Polypeptidsequenzen für die strukturellen Komponenten des Virions, das heißt für das Kapsid-Protein, C, sowie für mindestens zwei virale Envelope-Proteine (Flaviviren: prM, E, NS1; Pestiviren: E^{rns}, E1, E2; HCV: E1, E2). Die Strukturproteine werden in erster Linie von Signalpeptidasen der Wirtszelle aus dem Polyprotein generiert. Die Envelope-Proteine, die stark glykosiliert werden, assoziieren zu Homo- und Heterodimeren: Bei Pestiviren und HCV sind E2-E2- und E2-E1-Dimere dokumentiert. Die Funktion des löslichen pestiviralen Envelope-Proteins E^{rns} ist bisher unklar: Es besitzt eine Ribonuklease-Funktion und wird für die Infektion in Zellkultur nicht benötigt. Die Dimerisierung spielt beim Virus-Entry eine wichtige Rolle (für Review, siehe Lindenbach and Rice, 2001). Die Prozessierung des C-terminalen Nicht-Struktur(NS)-Polyproteins NS2A-NS2B-NS3-NS4A-NS4B-NS5 (bei Flaviviren) bzw. NS2-NS3 (bei Pestiviren NS2-3) - NS4A-NS4B-NS5A-NS5B (bei Pestiviren und bei HCV), wird bei allen drei Genera vorwiegend durch einen viralen Proteasekomplex katalysiert. Dieser besteht aus einer Serinprotease, die sich im aminoterminalen Bereich des NS3-Proteins befindet und einem Kofaktor (NS2B bei Flaviviren, NS4A bei Pestiviren und HCV). Bei Flaviviren generiert der NS3-NS2B-Komplex alle NS-Proteine, lediglich die Proteolyse zwischen NS4A-NS4B wird durch eine zelluläre Signalaseaktivität katalysiert. Bei Pestiviren und HCV spaltet NS3-4A das komplette NS-Polyprotein mit Ausnahme von NS2-NS3. Letztere Spaltung erfolgt bei HCV durch eine Autoprotease katalysiert, die an dieser Stelle lokalisiert ist (für Review, siehe Lindenbach and Rice, 2001). Beim Pestivirus BVDV ist das Ausmaß der proteolytischen Spaltung zwischen NS2 und NS3 interessanterweise mit einem zytopathischen Effekt der Virusinfektion verknüpft.

Weiterhin wurde gezeigt dass es sich bei NS5 (bei Flaviviren) bzw. NS5B (Pestiviren, HCV) um die virale RNA-abhängige RNA-Polymerase (RdRp) handelt. Die Funktionen von NS4B und NS5A sind bislang ungeklärt.

### 2.3. Genomreplikation der Flaviviridae

Entsprechend dem allgemeinen Replikations-Modell von Positiv-Strang RNA-Viren assoziiert im Zytoplasma der von *Flaviviridae* infizierten Wirtszelle in direkter funktioneller Kopplung an Translation und Proteolyse des Polyproteins ein funktioneller membrangebundener Replikationskomplex, der aus der viralen RNA sowie viralen und zellulären Faktoren besteht. Dieser Komplex katalysiert zunächst die Synthese einer geringen Anzahl komplementärer Minus-Strang RNA-Molekülen; diese dienen dann in einem zweiten Schritt als Matrize für die Synthese eines signifikanten Überschusses neuer Plus-Strang RNA-Genome.

In den vergangenen Jahren ist der RNA-Replikationsprozess besonders bei Pestiviren intensiv untersucht worden. Maßgebliche Gründe hierfür waren das bereits erwähnte Fehlen eines infizierbaren Zellkultursystems für HCV und der Modellcharakter von Pestiviren für HCV. Basierend auf Daten mit BVDV konnte kürzlich auch ein effizientes Replikationssystem für das HCV etabliert werden (Lohmann et al. 1999). Für Pestiviren stehen bereits seit geraumer Zeit infizierbare Zellen in Kultur zur Verfügung. Zudem wurden 1996 "infektiöse cDNA Konstrukte" für BVDV und CSFV konstruiert, von denen aus sich durch *in vitro-*Transkription komplette virale RNA-Genommoleküle gewinnen lassen. Nach Transfektion in Wirtszellen sind diese RNA-Moleküle in der Lage, den kompletten pestiviralen Lebenszyklus bis hin zur Synthese neuer Viruspartikel zu durchlaufen (Moormann et al., 1996; Meyers et al., 1996). Durch Mutagenese der cDNA können Mutationen gezielt in die viralen RNA-Transkripte eingeführt werden und deren Effekt auf den viralen Lebenszyklus bestimmt werden ("reverse Genetik").

Die identische Organisation von Pestivirus und HCV-Replikons ist ein eindeutiger Hinweis darauf, dass die generellen molekularen Mechanismen, die der viralen RNA-Replikation unterliegen, zwischen beiden Genera konserviert sind. Im Falle von HCV ist es aber weder mit Virusisolaten aus Patienten noch mit *in vitro*-transkribierter kompletter genomischer RNA (Pietschmann et al., 2002) gelungen, den viralen Lebenszyklus in Zellkultur zu reproduzieren. BeiUntersuchungen, die den Eintritt des Virus in die Zelle, das Uncoating des Genoms sowie die Maturierung und Sekretion von Nachkommenviren betreffen, ist man also dringend auf das Pestivirus-Modell angewiesen.

### 2.4. Virion-Assembly und Sekretion von Nachkommenviren

Die späten Schritte des viralen Lebenszyklus sind bei allen drei *Flaviviridae* Genera kaum untersucht. Man nimmt an, dass ein Anteil der neu synthetisierten Positiv-Strang RNA-Moleküle in "statu nascendi" mit Kapsid-Proteinen zu Nukleokapsid-Partikeln assoziiert. Diese gelangen durch Knospung (engl: Budding) in zytoplasmatische Vesikel des endoplasmatischen Retikulums. Da die Envelope-Proteine bei der Maturierung in der ER-Membran verbleiben, entstehen auf diese Weise die umhüllten Virionen, die die Envelope-Proteine in korrekter Orientierung enthalten. Die Vesikel fusionieren im Zuge der zellulären Sekretion mit der Plasmamembran, wodurch die Viruspartikel in das extrazelluläre Kompartiment freigesetzt werden (Lindenbach and Rice, 2001).

### 2.5. Das Problem der Heterogenität

Während der Vermehrung von *Flaviviridae* entstehen häufig Mutationen. Deshalb sind die entsprechenden Wirtsorganismen stets mit einer sehr heterogenen Population dieser Viren infiziert. Die Heterogenität beeinflusst die Pathogenese, die Virusresistenz, das Ansprechen auf die Therapie mit Interferonen (IFN) und antiviralen Substanzen (Nukleosidanaloge und andere) sowie die Erkennung infizierter Zellen durch das Immunsystem. Diese Tatsache untermauert, dass neue antivirale Strategien notwendig und sinnvoll sind.

### 2.6. Möglichkeiten der Therapie einer chronischen HCV-Infektion

Eine der wenigen Therapiemöglichkeiten einer chronischen HCV-Infektion ist die Behandlung mit Interferon (IFN) alpha und Derivaten. Wesentlicher Nachteil von IFN-Therapien ist, dass diese häufig mit negativen Nebenwirkungen assoziiert sind. Seit 1999 ist für die Therapie der chronischen HCV das Guanosinanalogon Ribavirin in Kombination mit Interferonen zugelassen. Die Wirkungsweise dieses Medikaments ist jedoch nur unvollständig geklärt. Ribavirin hat außerdem häufig eine Reihe von Nebenwirkungen, wobei die Ribavirininduzierte Hämolyse von besonderer Bedeutung ist. Neben Ribavirin werden weitere Nucleosidanaloga sowie eine Reihe von Substanzen unbekannter Wirkweise verwendet (für Review siehe Trautwein und Manns, 2001). Für alle gegenwärtig zugelassenen Medikamente gegen HCV-Infektion gilt, dass es eine hohe Zahl von Nonrespondern gibt (in der Regel etwa 30-40%). Die Erfolgsrate ist noch geringer bei Koinfektionen verschiedener Viren (z.B. dem humanen Immundefizienzvirus HIV und HCV oder HBV und HCV). Selbst bei klinisch erfolgreicher Behandlung werden alle viralen Reservoirs nur selten komplett eliminiert und können zu einer Reaktivierung der Infektion führen (Rehermann et al., 1996). Bei fehlendem Ansprechen auf die Therapie oder gar einer Reaktivierung der Virusreplikation können grundsätzlich nur neue Medikamente helfen, wie sie in dieser Erfindung beschrieben werden. Die Risiken des Einsatzes aller bisher eingesetzten antiviralen Medikamente sind noch nicht mit Sicherheit zu beurteilen. Nukleosidanaloga wie Ribavirin bergen prinzipiell die Gefahr, dass resistente Viren entstehen und dass es zu Mutationen im Wirtsgenom kommt, die Anlass zur Entstehung von Krebs geben könnten. Die im Rahmen der hier vorgestellte Erfindung genannten neuen Medikamente sind nicht mit diesen Risiken behaftet bzw., diese sind sehr viel unwahrscheinlicher oder nicht zu erwarten.

Folgende Patentschriften, welche die vorliegende Erfindung nicht unmittelbar betreffen, wurden veröffentlicht: ein Verfahren zur Bestimmung der Proteasom-Aktivität in biologischen Proben (WO 00/23614); die Verwendung von Proteasom-Inhibitoren als Mittel zur Behandlung von Krebs, Entzündungen und Autoimmunerkrankungen (WO 99/22729); die Verwendung von Inhibitoren des UPS als Mittel zur Behandlung von Entzündungen und Autoimmunerkrankungen (WO 99/15183). Zum Stand der Technik gemäß Artikel 54 (3) EPÜ zählt auch die Anmeldung WO 02/30455 A, veröffentlicht am 18.04.2002. Darin wird die Inhibition von Hepatitis-C-Viren (HCV) mittels Proteasom-Inhibitoren offenbart. HCV zählt zum Genus Hepacivirus der Familie Flaviviridae.

### Wesen der Erfindung

Der Erfindung liegt die Aufgabe zugrunde, Mittel zur Hemmung der Freisetzung und der Infektiosität von weiteren Mitgliedern der *Flaviviridae,* der Genera Flavivirus oder zur Verfügung zu stellen. Die Aufgabe wurde durch den Einsatz von mindestens einem Proteasom-Inhibitor gemäß den Merkmalen der Patentansprüche gelöst. Gemäß einer bevorzugten Ausführungsform der Erfindung können als Proteasom-Inhibitoren Substanzen eingesetzt werden, welche die Aktivitäten des zellulären Proteasom-Pathway hemmen, regulieren oder anderweitig beeinflussen. Es ist auch möglich, dass als Proteasom-Inhibitoren Substanzen eingesetzt werden, die speziell die enzymatischen Aktivitäten des kompletten 26S Proteasom-Komplexes und der freien, nicht mit regulatorischen Untereinheiten assemblierten 20S katalytisch aktiven Proteasom-Struktur beeinflussen. Diese Inhibitoren können entweder eine oder mehrere oder alle der drei hauptsächlichen proteolytischen Aktivitäten des Proteasoms (die Trypsin-, die Chymotrypsin- und die Postglutamyl-Peptid hydrolysierenden Aktivitäten) innerhalb des 26S- oder auch des 20S-Proteasom-Komplexes hemmen.

Eine Variante der Erfindung besteht darin, als Proteasom-Inhibitoren Substanzen einzusetzen, die von Zellen höherer Eukaryoten aufgenommen werden und nach Zellaufnahme mit der katalytischen beta-Untereinheit des 26S-Proteasoms in Wechselwirkung treten und dabei alle oder einzelne der proteolytischen Aktivitäten des Proteasom-Komplexes irreversibel oder reversibel blockieren.

Als eine weitere Form der Erfindung kommen Mittel zum Einsatz, welche die Aktivitäten der Ubiquitin-konjugierenden wie auch der Ubiquitin-hydrolysierenden Enzyme hemmen. Polyubiquitinierung gilt allgemein als ein Erkennungssignal für die Proteolyse durch das 26S-Proteasom, und Beeinflussung des Ubiquitin-Pathway kann ebenfalls die Aktivität des Proteasoms regulieren.

Erfindungsgemäß werden als Proteasom-Inhibitoren auch Substanzen eingesetzt, die in verschiedenen Formen *in vivo* oral, intravenös, intramuskulär, subkutan, in verkapselter Form mit oder ohne Zell-Spezifität-tragende Veränderungen oder anderweitig verabreicht werden, aufgrund der Anwendung eines bestimmten Applikations- und Dosis-Regimes eine geringe Zytotoxizität und/oder hohe Selektivität für bestimmte Zellen und Organe aufweisen, keine oder unbedeutende Nebenwirkungen auslösen, eine relativ hohe metabolische Halbwertszeit und eine relativ geringe Ausscheidungs(Clearance)-Rate im Organismus aufweisen.

Als Proteasom-Inhibitoren werden des weiteren Substanzen eingesetzt, die in natürlicher Form aus Mikroorganismen oder anderen natürlichen Quellen isoliert werden, durch chemische Modifikationen aus natürlichen Substanzen hervorgehen oder total-synthetisch hergestellt werden. Dazu gehören:
a) natürlich vorkommende Proteasom-Inhibitoren:
   - Epoxomicin und Eponemycin,
   - Aclacinomycin A (auch bezeichnet als Aclarubicin),
   - Lactacystin und dessen chemisch modifizierte Varianten, insbesondere die Zellmembranpenetrierende Variante "Clastolactacystein β-Lacton",
b) synthetisch hergestellte:
   - modifizierte Peptidaldehyde wie zum Beispiel N-carbobenzoxy-L-leucinyl-L-leucinyl-L-leucinal (auch bezeichnet als MG 132 oder zLLL), dessen Borsäure-Derivat MG232; N-carbobenzoxy-Leu-Leu-Nva-H (bezeichnet als MG115); N-Acetyl-L-Leuzinyl-L-Leuzinyl-L-Norleuzinal (bezeichnet als LLnL); N-carbobenzoxy-Ile-Glu(OBut)-Ala-Leu-H (auch bezeichnet als PSI);
   - Peptide, die C-terminal α,β-Epoxyketone (auch bezeichnet als Epoxomicin oder Eponemycin), Vinyl-sulphone (zum Beispiel Carbobenzoxy-L-Leucinyl-L-Leucinyl-L-Leucin-vinyl-sulfon oder 4-Hydroxy-5-iodo-3-nitrophenylactetyl-L-Leucinyl-L-Leucinyl-L-Leucin-vinyl-sulfon, auch bezeichnet als NLVS), Glyoxal oder Borsäure-Reste (zum Beispiel Pyrazyl-CONH(CHPhe)CONH(CHisobutyl)B(OH)₂), auch bezeichnet als "PS-431" oder Benzoyl(Bz)-Phe-boroLeu, Phenacetyl-Leu-Leu-boroLeu, Cbz-Phe-boroLeu); Pinacol-Ester - zum Beispiel Benzyloxycarbonyl (Cbz)-Leu-Leu-boroLeu-Pinacol-Ester - tragen;
      und
   - als besonders geeignete Verbindungen werden Peptide und Peptid-Derivate eingesetzt, welche C-terminal Epoxyketon-Strukturen tragen, hierzu zählen beispielsweise Epoxomicin (Molekülformel: C₂₈H₈₆N₄O₇) und Eponemycin (Molekülformel: C₂₀H₃₆N₂O₅);
   - chemisch modifizierte Derivate auf der Basis von natürlich vorkommenden , insbesondere ein β-Lacton-Derivat mit der Bezeichnung PS-519 (1R-[1S,4R,5S]]-1-(1-Hydroxy-2-methylpropyl)-4-propyl-6-oxa-2-azabicyclo[3.2.0]heptane-3,7-dione, Molekülformel: C₁₂H₁₉NO₄), welches sich von dem natürlichen Proteasom-Inhibitor Lactacystin ableitet;
   - bestimmte Dipeptidyl Borsäure-Derivate, insbesondere Verbindungen, welche sich von dem Pyranozyl-Phenyl-Leuzinyl-Borsäure-Derivat mit dem Namen "PS-341" (N-Pyrazinecarbonyl-L-Phenylalanin-L-leuzin-Borsäure, Molekülformel: C₁₉H₂₅BN₄O₄)
   - ableiten. Hierzu zählen weiterhin die Verbindungen "PS-273" (Morpholin-CONH-(CH-Naphthyl)-CONH-(CH-isobutyl)-B(OH)₂) und dessen Enantiomer PS-293, die Verbindung PS-296 (8-Quinolyl-sulfonyl-CONH-(CH-Napthyl)-CONH(-CH-isobutyl)-B(OH)₂); die Verbindung PS-303 (NH₂(CH-Naphthyl)-CONH-(CH-isobutyl)-B(OH)₂); die Verbindung PS-321 (Morpholin-CONH-(CH-Napthyl)-CONH-(CH-Phenylalanin)-B(OH)₂); die Verbindung PS-334 (CH₃-NH-(CH-Naphthyl-CONH-(CH-Isobutyl)-B(OH)₂); die Verbindung PS-325 (2-Quinol-CONH-(CH-homo-Phenylalanin)-CONH-(CH-isobutyl)-B(OH)₂); die Verbindung PS-352 (Phenyalanin-CH2-CH2-CONH-(CH-Phenylalanin)-CONH-(CH-isobutyl)1-B(OH)₂); die Verbindung PS-383 (Pyridyl-CONH-(CHpF-Phenylalanin)-CONH-(CH-isobutyl)-B(OH)₂. Alle diese Verbindungen wurden bereits beschrieben, unter anderem in Adams et al. (1999).

Als besonders geeignete Verbindungen haben sich, neben Epoxomicin und Eponemycin, die Proteasom-Inhibitoren PS-519, PS-341 und PS-273 (Millennium Pharmaceuticals Inc., Cambridge, MA 02139, USA) erwiesen. Diese Proteasom-Inhibitoren sind sehr potent, sehr spezifisch für das Proteasom, blockieren keine anderen zelluläre Proteasen und haben daher so gut wie keine Nebenwirkungen. Die Proteasom-Inhibitoren PS-341 und PS-519 wurden außerdem sowohl in Tiermodellen für vorklinische als auch in Menschen (Krebspatienten) für klinische Studien getestet.

Die erfindungsgemäß eingesetzten Mittel sind dadurch gekennzeichnet, dass sie sich zur Hemmung der Freisetzung, Reifung und Replikation von Flavivirus-bedingtem Fieber, von Hämorrhagien, Leukopenie, Thrombozytopenie, von Durchfallerkrankungen, Encephalitiden sowie von Pestivirus-verursachten Krankheiten eignen.

Im Rahmen der vorliegenden Erfindung wird überraschenderweise festgestellt, dass Proteasom-Inhibitoren frühe oder späte Prozesse im Lebenszyklus des BVDV Pestivirus und des West-Nil-Flavivirus hemmen. Die intrazelluläre Replikation von BVDV- und HCV-RNA-Replikons wird dagegen von den Proteasom-Inhibitoren nicht oder kaum beeinflusst. Spezifisch wurde beobachtet, dass sich die erfindungsgemäße Verwendung von Proteasom-Inhibitoren dazu eignet, die Produktion von infektiösen Virionen in mit BVDV- und West-Nil-infizierten Zellen weitgehend oder vollkommen zu unterbinden. Nach Behandlung von BVDV- und West-Nil-Virus-produzierenden Zellen mit Proteasom-Inhibitor tritt eine nahezu vollständige Reduktion der Infektiosität der freigesetzten Virionen ein. In Folge dieser neuartigen Aktivitäten können Proteasom-Inhibitoren bei verschiedenen Mitgliedern der *Flaviviridae*-Familie die Neuinfektion von Wirtszellen und damit die Ausbreitung einer Infektion *in vivo* unterdrücken.

Die Daten mit BVDV und West-Nil-Virus rechtfertigten ferner den Anspruch, dass Proteasom-Inhibitoren unter Verwendung der neuartigen anti-viralen Wirkung für die Behandlung weiterer *Flaviviridae* wie zum Beispiel Dengue-Fieber-Virus oder Frühsommer-Meningoenzephalitis (FSME) Virus eingesetzt werden können.

Die Proteasom-Inhibitoren können auch in Kombination mit sonstigen Therapieschemata eingesetzt werden, z.B. Interferon alpha/beta/gamma und Varianten hiervon (zum Beispiel pegylierte Interferone), Interleukine, Nukelosidanaloga (Lamivudine, Cidovir, Ribavirin und andere), Steroide, Plasma-Austausch, Thymosin alpha 1, Impfstoffe, passive und aktive Vakzinierung, therapeutische und prophylaktische Vakzinierung, Glycyrrhizin, Stammzelltransplantation, Organtransplantationen, Nahrungstherapie, Immunsuppressiva, Cyclosporine und Derivate hiervon, Amanditin und Derivate, Interleukine und andere Cytokine, nicht Proteasom-selektive Protease-Inhibitoren, Azathioprin, Hämodialyse.

Die erfindungsgemäße Verwendung von Proteasom-Inhibitoren besteht in der Hemmung des Entry/Internalisierung- und Uncoating-Prozesses von *Flaviviridae* sowie in der Hemmung der Assemblierung, Reifung und Freisetzung von Nachkommenviren. Die Verwendung zur Hemmung der Vermehrung von *Flaviviridae* erfolgt nach den Mechanismen
a) Blockierung/Reduktion der Assemblierung und Freisetzung von neuen Virionen
b) Blockierung/Reduktion der Infektiosität der freigesetzten Virionen
c) Blockierung/Reduktion der Infektionsausbreitung in kultivierten Zellen.

Dies impliziert, dass Proteasomeninhibitoren die Ausbreitung von Nachkommenviren in infizierten Organen inhibieren.

Eine weitere Anwendung besteht darin, dass Proteasom-Inhibitoren die posttranslationale Modifikation der Virus-Strukturproteine verändern und somit die Freisetzung und Infektiosität von Flaviviridae herabsetzen oder blockieren.

Eine weitere Verwendung von Proteasom-Inhibitoren besteht in der Behandlung von mit Flavivirus infizierten Personen, also zum Beispiel Personen, die akut an West-Nil-, Gelbfieber, Dengue-Fieber (7-Tage-Fieber oder Dengue Hämorrhagischem Fieber) oder Arbovirus-Encephalitis erkrankt sind. Proteasom-Inhibitoren können auch hier zur Vorbeugung einer Virusinfektion bei Risikopersonen wie Ärzten oder Reisenden in hochendemische Gebiete für West-Nil-Virus, Dengue-Fieber-Virus, Gelbfieber-Virus oder FSME-Virus eingesetzt werden.

Ein weiteres Anwendungsbeispiel ist die Behandlung von Pestivirus-infizierten Stalltieren mit Proteasom-Inhibitoren.

Zur Lösung der Aufgabe wurden im Rahmen der Erfindung molekularvirologische, biochemische, immunbiologische und elektronenmikroskopische Studien an Zellen durchgeführt, die mit verschiedenen Vertretern der *Flaviviridae* infiziert oder mit viralen RNA-Molekülen transfiziert wurden. Erfindungsgemäß wurden die durch Proteasom-Inhibitoren ausgelösten Defekte durch folgende Mittel und Verfahren bestimmt: (i) Viruspräparationen von BVDV (Stamm CP7) und West-Nil-Flavivirus mit definiertem Titer infektöser Einheiten; (ii) Virus-Endpunkt-Titrationsverfahren durch mikroskopische Detektion infektiöser viraler Partikel über Plaque-Formation oder Immunfärbeverfahren; (iii) cDNA-Konstrukte zur Herstellung subgenomischer BVDV- und HCV-RNA-Replikon durch *in vitro*-Transkription; (IV) RNase-Protektionsverfahren zur Detektion/Quantifizierung viraler RNA-Moleküle; (v) Immunofluoreszenz-Tests zur Bestimmung der Replikationsfähigkeit viraler RNA-Moleküle bzw. zur Bestimmung der Ausbreitung einer Infektion; (vi) Elektronenmikroskopische Verfahren zur Untersuchung der Morphologie viraler Partikel während und nach dem Infektionsvorgang, (vii) Western-Blot Studien und Immunopräzipitationsverfahren an BVDV- und HCV-Proteinen.

Die Prinzip-Lösung der Aufgabe wird an den Beispielen von BVDV und West-Nil-Virus gezeigt, d.h. zwei charakteristischen Vertretern der Genera *Pestivirus* bzw. *Flavivirus* der *Flaviviridae-Familie.* In Kontrollversuchen wurde zunächst dargestellt, dass eine Vorbehandlung der Target-Zellen (MDBK-Zellen für BVDV, BHK-21-Zellen für West-Nil-Virus) mit nicht-zytotoxischen Konzentrationen verschiedener Substanzklassen von Proteasom-Inhibitoren keinen Einfluss auf eine nachfolgende Infektion der Viren hat. Ebenso wurde gezeigt, dass eine Vorbehandlung von BVD-Viren und West-Nil-Viren mit nicht-toxischen Konzentrationen verschiedener Proteasom-Inhibitoren keinen Einfluss auf deren Infektiosität hat.

Im Rahmen der Erfindung wird erstmalig gezeigt, dass die Behandlung von bereits infizierten Zellen mit verschiedenen Proteasom-Inhibitoren die Produktion von infektiösen Viruspartikeln signifikant hemmt: Durch Virus-Endpunkt-Titration auf nicht-infizierten Zellen wurde erfindungsgemäß festgestellt, dass die Anzahl freigesetzter infektiöser Nachkommenviren aus infizierten Zellen, die mit Proteasom-Inhibitoren behandelt wurden, im Vergleich zu Mock-behandelten Zellen um mehrere log-Stufen sinkt. Neben der drastischen Reduktion der Anzahl an Nachkommenviren verursacht die Behandlung mit Proteasom-Inhibitoren auch eine deutliche Abnahme der spezifischen Infektiosität der Nachkommenviren. Die spezifische Infektiosität wurde über die Anzahl der in den Kulturüberstand infizierter Zellen freigesetzten Virusgenome (gemessen durch quantitative RNase-Protektion) im Verhältnis zum Titer der infektiösen Einheiten bestimmt. In Immunfluoreszenzstudien wurde deutlich, dass sowohl die RNA-Replikation als auch die Ausbreitung der Infektion in Zellkultur unter dem Einfluss verschiedener Proteasom-Inhibitoren stark reduziert ist. Die Reduktion der RNA-Replikation in den infizierten und mit Proteasom-Inhibitoren behandelten Zellen wurde erfindungsgemäß durch Messung der RNA-Replikationsprodukte via RNase-Protektion bestätigt.

Weiterhin wurde durch die Verwendung von Western-Blotkinetik-Studier festgestellt, dass die Virusfreisetzung von BVDV-infizierten Zellen signifikant reduziert ist, wenn diese Zellen kurze Zeit nach einer Infektion mit Proteasom-Inhibitoren behandelt wurden.

In überraschendem Gegensatz dazu wurde in einem weiteren Teil der Erfindung festgestellt, dass die verschiedenen Substanzklassen von Proteasom-Inhibitoren nur geringe Effekte auf die Replikation von persistent in MDBK- bzw. Huh-7-Zellen transfizierte BVDV- und HCV-RNA-Replikons haben. Diese Ergebnisse zeigten, dass Proteasom-Inhibitoren nicht oder nur in geringem Maße mit folgenden durch virale Elemente und Faktoren katalysierten Vorgängen interferieren: (i) Mit der Translation der viralen RNA über das in der 5'-UTR enthaltene IHRES (interne Ribosomen-Eintrittsstelle) -Element; (ii) Mit der von den viralen Proteasekomplexen NS3/NS4A bzw. NS2-NS3 mediierten Proteolyse des Nicht-Strukturpolyproteins; (iii) Mit der von den viralen Proteinen NS3, NS4A, NS4B, NS5A, NS5B (und hypothetischen Zwischenprodukten des Nicht-Strukturpolyproteins) und den nicht-translatierten Regionen des Genoms katalysierten Replikation der viralen RNA. Die Targets klassischer anti-viraler Substanzen, d.h. die IRES, die viralen Proteasen, die NS3-ATPase/RNA-Helikase sowie die NS5/NS5B-RdRp (siehe Einleitung), werden also von Proteasom-Inhibitoren nicht oder nur geringfügig gehemmt. Die drastische Verminderung der Sekretion infektiöser Nachkommenviren bei Behandlung infizierter Zellen mit Proteasom-Inhibitoren kann demnach nicht oder nicht ausschließlich auf die zu beobachtende Verminderung der RNA-Replikationsrate zurückgeführt werden, sondern muss folglich Vorgänge betreffen, die das Virus-Entry und/oder das Uncoating und/oder die Assemblierung und/oder Sekretion von Nachkommenviren betreffen.

Diese Hypothese wurde erfindungsgemäß durch Virus-Zell-Adhäsionsexperimente und durch elektronenmikroskopische Untersuchungen untermauert. So wurde einerseits festgestellt, dass Nachkommenviren, die aus infizierten Zellen unter Proteasom-Inhibitor-Behandlung freigesetzt werden, bedeutend ineffizienter an Target-Zellen adhärieren. Weiterhin sind nach Behandlung infizierter Zellen mit Proteasom-Inhibitoren im Vergleich zu Mock-behandelten Zellen Unterschiede in der Anzahl der über Elektronenmikroskopie generell in den Zellen zu detektierenden Viruspartikel, Unterschiede im Verhältnis von kompletten zu nicht-kompletten Virionen sowie Unterschiede in der Morphologie der sekretierten Nachkommenviren festzustellen.

In einem weiteren Teil der Erfindung wird gezeigt, dass in Gegenwart von Proteasom-Inhibitoren signifikante Veränderungen in der post-translationalen Modifikation der BVDV- und HCV-Strukturproteine, der Prozessierung des Strukturprotein-Polyproteins und der Dimerisierungsfähigkeit der Envelope-Proteine auftreten. Diese Veränderungen könnten damit kausal die Effekte von Proteasom-Inhibitoren auf den viralen Lebenszyklus, wie sie bei Pestiviren und Flaviviren festgestellt wurden, erklären.

Erfindungsgemäß wird damit gezeigt, dass der hemmende Effekt von Proteasom-Inhibitoren auf die Virusvermehrung von verschiedenen Vertretern der *Flaviviridae*-Familie auf folgenden Mechanismen beruht:
1. Blockierung/Reduktion des Entry- und/oder Uncoating-Prozesses der viralen RNA.
2. Blockierung / Reduktion des Assembly und / oder der Sekretion von Nachkommenviren.
3. Blockierung/Reduktion der Infektiosität von freigesetzten Virionen.
4. Blockierung/Reduktion der Infektionsausbreitung.

Diese Mechanismen implizieren, dass Proteasomen-Inhibitoren die Infektionsausbreitung von verschiedenen Mitgliedern der *Flaviviridae*-Familie auch in infizierten Organen blockieren. Die ausgeprägten Homologien von Pestiviren und Hepatitis-C-Viren rechtfertigt ferner die Annahme, dass Proteasomeninhibitoren eine ähnliche Wirkung bei HCV-Infektionen haben.

Aufgrund dieser neuartigen Behandlungsmethode können daher vielfältige therapeutische Effekte durch den Einsatz von Proteasom-Inhibitoren bei Infektionen mit Mitgliedern der *Flaviviridae*-Familie ausgelöst werden. Neben den ausgeführten Vorteilen besteht ein weiterer wesentlicher Vorteil darin, dass mit dieser Strategie zelluläre Faktoren getroffen werden, welche für den Lebenszyklus von *Flaviviridae* essentiell sind, die jedoch eine im Vergleich zu viralen Faktoren erheblich höhere genetische Stabilität haben. Aufgrund dieser genetischen Stabilität der Zielstruktur dieser neuartigen anti-viralen Strategie ist mit dem Auftreten von Resistenz-Erscheinungen, wie sie für viele der bislang bekannten Inhibitoren von RNA-Viren beobachtet werden konnten, nicht zu rechnen.

Die Merkmale der Erfindung gehen aus den Elementen der Ansprüche und aus der Beschreibung hervor, wobei sowohl einzelne Merkmale als auch mehrere in Form von Kombinationen vorteilhafte Ausführungen darstellen, für die mit dieser Schrift Schutz beantragt wird. Das Wesen der Erfindung liegt in der Verwendung bekannter Mittel zu einem neuen Zweck und in einer Kombination bekannter Elemente - den Proteasom-Inhibitoren - und einer neuen Wirkung - ihrem Einsatz zur Behandlung von Flaviviridae-Infektionen - die in ihrer neuen Gesamtwirkung einen Vorteil und den erstrebten Erfolg ergeben, der darin liegt, dass nunmehr Mittel zur Vorbeugung, Behandlung, Therapie und Hemmung der von verschiedenen Mitgliedern der Virusfamilie Flaviviridae (Genera: Flavivirus, Pestivirus) verursachten Krankheiten beim Menschen und bei Tieren zur Verfügung stehen.

In einer weiteren Ausführungsform der Erfindung wird ebenfalls dargestellt, dass durch den Einsatz von Proteasom-Inhibitoren die Produktion von BVDV von infizierten Zellen verhindert werden kann. Zur Lösung dieser Aufgabe werden kultivierte Zellen mit BVDV (Stamm CP7, NADL) infiziert und nach Ausbreitung der Infektion in der Kultur im Verlauf einer Zeitkinetik BVDV-Virionen isoliert und deren Menge biochemisch quantifiziert. Damit wird bewiesen, dass unter Verwendung verschiedenster Proteasom-Inhibitoren die Produktion von Virionen der Flaviviridae-Familie in einer infizierten Zelle gehemmt werden kann.

Das in der Erfindungsbeschreibung dargestellte Prinzip der Verwendung von Proteasom-Inhibitoren zur Blockierung einer Infektion mit *Flaviviridae* ist neuartig in Hinsicht auf die Verwendung einer bereits bekannten Substanzklasse (den Proteasom-Inhibitoren) für eine neue Aktivität, welche sich in folgenden therapeutischen Konzepten zusammenfassen lässt:

die Blockierung der Produktion von infektiösen *Flaviviridae* und damit die Verhinderung der Infektionsausbreitung *in vivo.*

Gleichzeitig ist die Verwendung von Proteasom-Inhibitoren auch neuartig hinsichtlich des Anwendungsprinzips. Bislang sind keine Substanzen/ Prinzipien/ Methoden bekannt, welche vorwiegend frühe oder späte Prozesse der Replikation von *Flaviviridae,* speziell die Freisetzung von infektiösen Virionen beeinflussen. Weiterhin ist neu, dass die Verwendung von Proteasom-Inhibitoren zur Blockierung des Lebenszyklus von *Flaviviridae* führt. Neuartig ist die bei der Applikation von Proteasom-Inhibitoren erheblich geringere Wahrscheinlichkeit der Entstehung von Resistenzmechanismen im Vergleich zu bisherigen anti-viralen Methoden, die bei der Behandlung von RNA-Virus-Infektionen eingesetzt werden. Dies ist begründet durch die Tatsache, dass Proteasom-Inhibitoren weniger essentielle Komponenten des Virus direkt treffen, als vielmehr auf zelluläre Funktionen wirken, die offenbar für die Viriongenese wichtig sind. Die ist spezifisch messbar an den Effekten die Proteasom-Inhibitoren auf die Modifikation und Prozessierung sowie das Assoziationsverhalten der Virus-Strukturproteine.

Neuartig ist weiterhin das Prinzip der Wirkung von Proteasom-Inhibitoren, die zwar nicht die Initiation der Virusinfektion, wohl aber die Produktion von infektiösen Viruspartikeln von bereits mit *Flaviviridae* infizierten Zellen verhindern. Dadurch wird wesentlich die Menge an infektiösen Virionen (Viruslast) und somit die Infektionsausbreitung *in vivo* reduziert.

Aus der Summe dieser neuartigen Mechanismen lässt sich feststellen, dass die verminderte Freisetzung von noch dazu wenigen oder gar nicht infektiösen Viruspartikeln im Netto-Effekt im Falle einer *in vivo*-Anwendung von Proteasom-Inhibitoren die Menge an infektiösen Virionen in einem infizierten Organismus verringern sollte. Die Anwendung von Proteasomen-Inhibitoren allein oder in Kombination mit bereits angewendeten anti-viralen Therapien ist besonders attraktiv.

Die Erfindung soll anhand von Ausführungsbeispielen näher erläutert werden, ohne auf diese Beispiele beschränkt zu sein.

### Ausführungsbeispiele

### Beispiel 1:

### Die Behandlung von Flaviviridae-infizierten Zellkulturen mit moderaten Konzentrationen von Proteasomen-Inhibitoren reduziert drastisch die Freisetzung und Ausbreitung infektiöser Nachkommenviren.

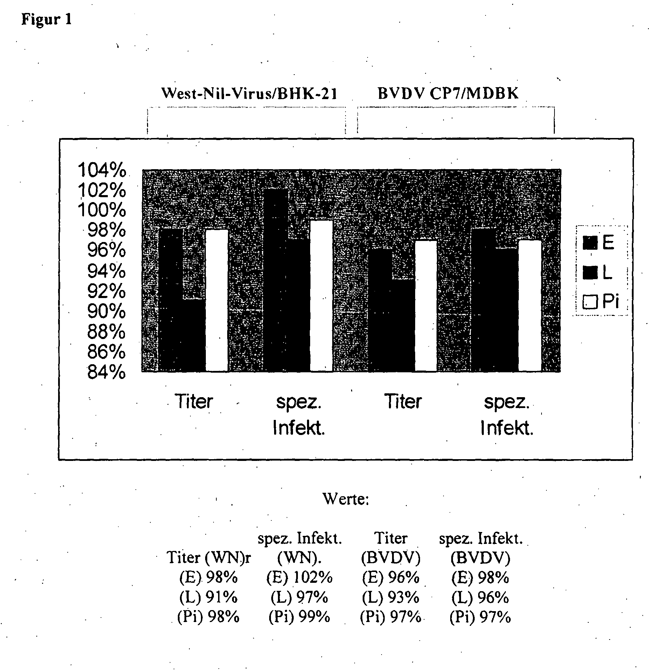

In einem Kontrollexperiment sollte zunächst getestet werden, welchen Effekt eine Vorbehandlung der Zellen mit Proteasomen-Inhibitoren auf eine Infektion mit verschiedenen *Flaviviridae-*Mitgliedern hat. Dazu wurden nicht-infizierte MDBK-Zellen und BHK-21-Zellen in Parallel-Ansätzen für unterschiedliche Zeiträume in Zellkulturmedium gehalten, das mit verschiedenen PI (Konzentration 10 nM) versetzt wurde. Die Zeitintervalle dieser Behandlung reichten dabei von 1-8 Stunden. Anschließend wurden die Zellen mit PBS gewaschen und nachfolgend mit einem geringen Volumen an Pestivirus (BVDV CP7) oder Flavivirus (West-Nil-Virus)-haltigem Kulturmedium bei einer MOI von 1-5 versetzt (MOI=multiplicity of infection=Anzahl der pro Zelle eingesetzten infektiösen Viruspartikel; diese war zuvor durch Virustitration ermittelt worden). Nach einer Infektionsphase von etwa einer Stunde bei 37°C wurde überschüssiges und/oder nicht infektiöses Virus durch einen Waschschritt mit PBS entfernt und die infizierten Zellen mit frischem Kulturmedium weiter gezüchtet. Nach einem Replikationszyklus, d.h. etwa 12 Stunden (West-Nil-Flavivirus) bzw. 30 Stunden (BVDV) wurde der virushaltige Kulturüberstand der Zellen abgenommen. Da es sich bei BVDV CP7 und West-Nil-Virus um zytopathische (zp) Viren handelt, ließ sich zu diesen Zeitpunkten in der Regel ein zytopathischer Effekt der Virusinfektion (ZPE) bei den infizierten Zellen feststellen. Der ZPE kann lichtmikroskopisch durch das Abrunden, Schrumpfen und Ablösen infizierter Zellen von der Kulturplatte festgestellt werden. Darüber hinaus wurde in parallel durchgeführten Immunofluoreszenztests mit spezifischen Antikörpern gegen virale Proteine (gemessen wurde dabei ausschließlich die replikationsabhängige Synthese dieser Proteine) gezeigt, dass zu diesen Zeitpunkten in praktisch allen Zellen virale Replikation festgestellt werden kann (Standardprotokoll; siehe auch Behrens *et al.,* 1998). Die Anzahl der im Kulturmedium enthaltenen infektiösen Viruspartikel wurde über eine Endpunkt-Titration (Standardprotokoll) auf nicht-infizierten BHK (West-Nil-Flavivirus) bzw. MDBK- (BVDV)-Zellen festgestellt. Aufgrund des zytopathischen Charakters beider Viren ließ sich der Titer durch die Formation von Plaques im Zellrasen mikroskopisch oder über Immunfärbeverfahren eines der viralen Proteine (Standardprotokolle) ermitteln. Zur Bestimmung der im Zellkulturüberstand insgesamt enthaltenen relativen Anzahl an Virusgenomen wurde ein definiertes Volumen des Zellkultur-Überstandes auf eine Endkonzentration von 0.5% (m/v) SDS (Sodium Dodecylsulfat), 150 mM NaCl und 50 mM Tris/Cl pH 7.5 gebracht und die enthaltenen Proteine durch Zugabe von 20µg/ml des Enzyms Proteinase K (PK) hydrolysiert. Die verbliebenen Nukleinsäuren wurden durch eine Phenol- und eine nachfolgende Chloroform-Extraktion gereinigt und durch Ethanol präzipitiert (Standard-Protokolle). Die in dem Zell-Überstand enthaltene virale RNA wurde durch ein von Behrens *et al.* (1998) und Grassmann *et al.* (1999) entwickeltes spezielles RNase-Protektionsverfahren quantifiziert. Das generelle Prinzip dieser Detektionsmethode beruht auf dem Einsatz kurzer durch Transkription *in vitro* (Standardprotokoll) hergestellter [³²P]-markierter RNA-Moleküle ("Probes"), die eine zu den viralen RNA-Molekülen komplementäre ("anti-sense") Orientierung aufweisen. Nach Hybridisierung der Proben an die virale RNA werden durch Umsetzung mit den einzelstrang-spezifischen Ribonukleasen A und T1 nicht gepaarte Bereiche der RNA verdaut und die Anzahl der doppelsträngigen ("RNase-protegierten") viralen RNA-Moleküle über die Messung der gleichfalls protegierten, markierten Probe-Moleküle quantifiziert (Grassmann *et al.,* 1999). Durch Bildung des Verhältnisses der insgesamt in einem bestimmten Volumen Zellüberstand enthaltenen Virusgenome und der durch Titration ermittelten Anzahl der infektiösen Nachkommenviren wurde die spezifische Infektiosität der neu assemblierten Nachkommenviren ermittelt. In Fig. 1 ist gezeigt, dass eine Vorbehandlung der Wirtszellen mit verschiedenen PI einen verhältnismäßig geringen Effekt auf eine nachfolgende Virusinfektion hat: Sowohl der Titer als auch die spezifische Infektiosität von Nachkommenviren sind bei einer Flavivirus und Pestivirus-Infektion, die nachfolgend auf eine Behandlung der entsprechenden Wirtszellen mit Proteasom-Inhibitoren durchgeführt wurde, im Vergleich zu einer Kontrolle mit Mock-behandelten Zellen geringfügig oder nicht verändert.

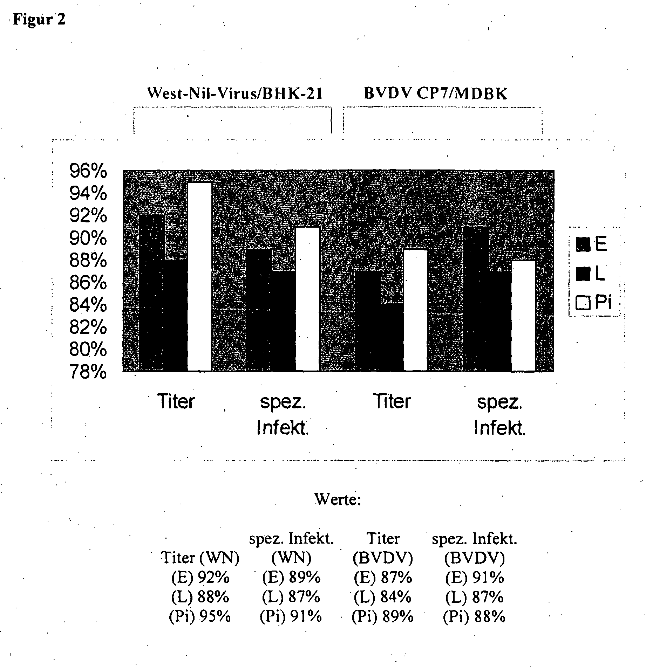

In einem weiteren Kontrollexperiment wurde überprüft, ob eine direkte Behandlung von *Flaviviridae*-Viruspartikeln mit Proteasom-Inhibitoren einen Einfluss auf die Infektiosität der Viren hat. Dazu wurden Kulturmedium-Überstände infizierter Zellen, die eine definierte Anzahl an West-Nil-Flavivirus bzw. BVDV-Viruspartikeln enthielten (diese wurde zuvor durch Virustitration ermittelt), mit verschiedenen PI (Konzentration wie in den oben beschriebenen Experimenten 10nM) versetzt und für 24 Stunden bei 37°C inkubiert. Es wurde festgestellt (Fig. 2), dass durch die Behandlung des Virusüberstandes mit Proteasom-Inhibitoren die Anzahl an infektiösen Einheiten im Vergleich zu einer unbehandelten Kontrolle nicht wesentlich reduziert wurde.

Damit kann folgendes festgestellt werden. 1. Die Behandlung von Viruspartikeln verschiedener Vertreter der *Flaviviridae* mit Proteasomen-Inhibitoren hat keinen oder nur einen geringen Einfluss auf die Infektiosität dieser Virus-Partikel. 2. Die Vorbehandlung der Zellen für maximal 8 Stunden mit Proteasomen-Inhibitoren hat keinen Einfluss auf eine sich der Behandlung anschließende Infektion der Zellen durch *Flaviviridae;* d.h., die für die Virusinfektion wichtigen zellulären Strukturen an der Zellmembran werden durch die Proteasom-Inhibitoren nicht oder kaum beeinflusst.

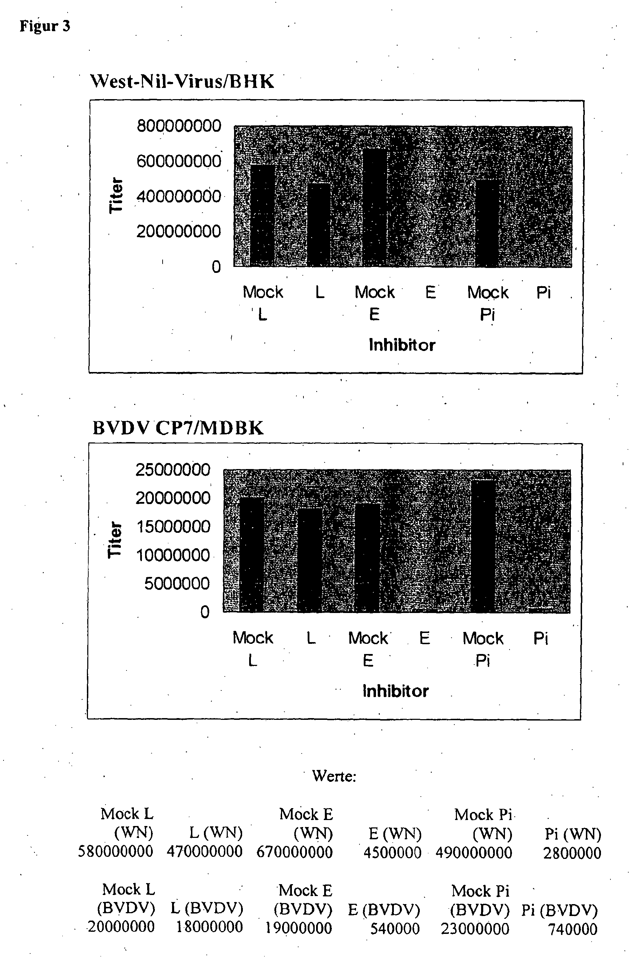

Um den Einfluss von Proteasomen-Inhibitoren auf die sich dem unmittelbaren Infektionsvorgang anschließenden Ereignisse einer *Flaviviridae-*Infektion wie Virus-Entry/Internalisierung, Uncoating, RNA-Replikation sowie Assembly und Sekretion sowie Infektiosität von Nachkommenviren (siehe Einleitung) zu untersuchen, wurden BHK- und MDBK-Zellen mit dem West-Nil-Flavivirus bzw. mit dem BVDV in einer Reihe von Parallel-Ansätzen in der oben genannten Weise infiziert. Nach dem Waschen der infizierten Zellen mit PBS wurden diese in frischem Medium aufgenommen, das mit 10 nM verschiedener PI versetzt wurde. Nach 12 (West-Nil-Flavivirus) bzw. 30 Stunden Inkubationszeit bei 37°C wurde der Kulturüberstand in der beschriebenen Weise gewonnen. Die Zellen wurden im Anschluss an den genannten Infektionszeitraum mit einem Rubber-Policeman von der Kulturplatte abgekratzt, in PBS gewaschen und für die weitere Verwendung auf Eis gehalten oder eingefroren. Die Anzahl neu assemblierter Nachkommenviren bzw. deren spezifische Infektiosität wurde über Virustitration und das genannte RNase-Protektionsverfahren aus dem Kulturüberstand ermittelt. Zur Messung der intrazellulären Replikation wurde die virale RNA aus den infizierten Zellen gewonnen. Dazu wurden die Zellen in einem Lysepuffer (50 mM Tris/Cl pH 8, 100 mM NaCl, 5 mM MgCl₂, 0.5% v/v NP40) für 10 Minuten auf Eis aufgeschlossen, die Zellkerne über eine Zentrifugation bei 1000 g abgetrennt und die virale RNA aus dem Zytoplasma durch PK-Verdau, Phenol- und Chloroformextraktion sowie Ethanol-Präzipitation gewonnen. Die Anzahl der in den Zellen enthaltenen viralen RNA-Replikationsprodukte (Minus-Strang RNA Intermediat und neu synthetisierte Plus-Strang RNA) wurde durch RNase-Protektion quantifiziert. Um die bei verschiedenen Infektionsexperimenten auftretende Variation der Zellzahl auszugleichen, wurde folgendermaßen verfahren: Durch Messung der optischen Dichte (OD₂₆₀; Standard-Protokoll) wurde die Gesamtmenge der extrahierten zytoplasmatischen RNA bestimmt (in früheren Arbeiten konnte gezeigt werden, dass auch bei effizient replizierenden Viren nur ein Bruchteil der Gesamt-Zytoplasma-RNA virale RNA ist) und eine definierte Quantität dieser RNA in den RNase-Protektionsexperimenten eingesetzt. In Fig. 3-5 ist gezeigt, dass sowohl die RNA-Replikationsrate als auch die Anzahl an freigesetzten Nachkommenviren infolge der Behandlung der Zellen mit PI nach der Infektion signifikant herabgesetzt war. Dabei konnten allerdings deutliche Unterschiede zwischen den PI beobachtet werde: So war der Effekt von Lactacystin gering, während eine Behandlung mit Epoxomycin oder einem Proteasominhibitor Pi maximale Wirkung zeigte, d.h. eine Reduktion der RNA-Replikationsrate um ca. einen Faktor von 5-10 und Reduktion der Anzahl freigesetzter Nachkommenviren um mehrere Log-Stufen. Es konnte auch eine deutliche Reduktion der spezifischen Infektiosität, d.h. des Verhältnisses der Anzahl der im Überstand detektierbaren RNA-Genome zu der Anzahl der im Titratiönstest ermittelten infektiösen Einheiten (um etwa einen Faktor 50-100) ermittelt werden.

Um zu testen, ob die festgestellte geringere Infektiosität der Nachkommenviren durch eine verminderte Adhäsionsfähigkeit der Viruspartikel an die Zielzelle bedingt ist, wurde folgendes Experiment durchgeführt: BHK- und MDBK-Zellen wurden in parallelen Ansätzen mit West-Nil-Flavivirus- bzw. BVDV-haltigen Überständen versetzt, die aus PI-behandelten bzw. nicht-behandelten Infektionsexperimenten gewonnen worden waren. Wichtig war in diesem Zusammenhang, dass eine jeweils identische MOI bei der Infektion eingesetzt wurde. Nach einer Inkubationsphase bei 4°C für eine Stunde wurde ein Teil der Zellen für eine Stunde auf 37°C gebracht, der andere Teil der Zellen wurde sofort weiter aufgearbeitet. Die Zellen wurden durch Abkratzen von der Platte gewonnen, und aus den jeweiligen Ansätzen wurde anschließend die Gesamt-RNA durch PK-Verdau und Phenol- und Chloroform-Extraktion gewonnen. Das Verhältnis der bei 4°C an die Zellen adhärierten und bei 37°C verbliebenen/internalisierten viralen RNA-Genome wurde anschließend über eine RNase-Protektion quantifiziert. Dabei wurde deutlich, dass die Viruspartikel, die als Nachkommenviren aus Proteasom-Inhibitor behandelten Zellen sekretiert wurden, erheblich weniger infektiös sind als Viren, die aus unbehandelten Zellen gewonnen wurden.

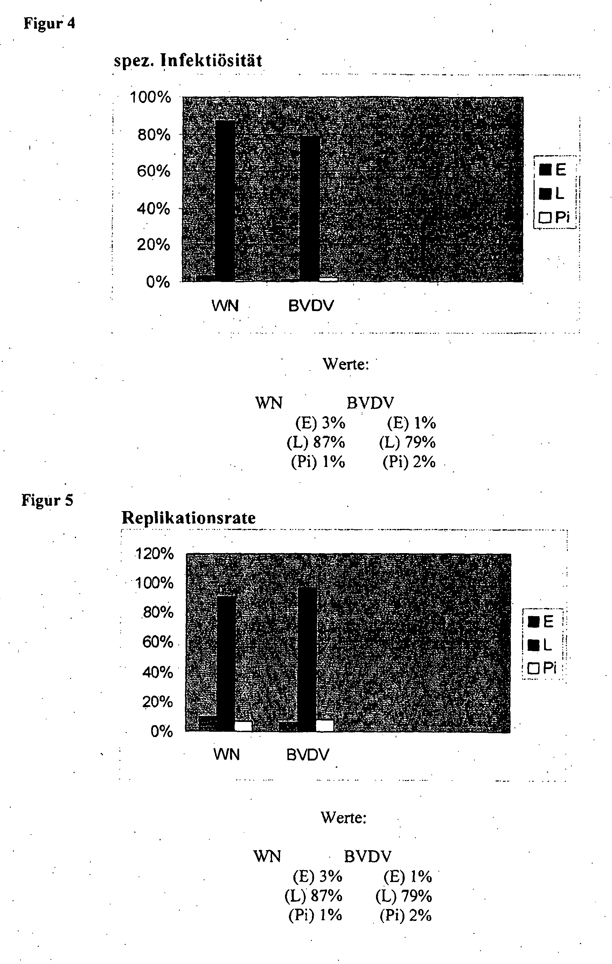

Konsequenterweise ließ sich in Zellkulturen von mit Flavi-, und Pestiviren infizierten Zelllinien in Gegenwart von Proteasomen-Inhibitoren eine deutliche Verminderung der Infektionsausbreitung feststellen. Letztere Tatsache ließ sich durch Immunfluoreszenztests besonders eindrucksvoll belegen: Während bei einer Infektion unter Mock-Behandlung typische Plaqueformationen der infizierten Zellen unter dem Mikroskop ausgemacht werden - dies ist eine Folge der horizontalen Ausbreitung der Virusinfektion - war dies unter PI-Behandlung nicht der Fall (Fig. 5).

Zusammengefasst verdeutlichen diese Experimente, dass die Behandlung infizierter Zellen mit Proteasom-Inhibitoren zu einer Blockierung/Reduktion der Replikationsrate der viralen RNA in infizierten Zellen, zu einer Blockierung/Reduktion der Freisetzung von neuen Virionen und zu einer Blockierung/Reduktion der Infektiosität von freigesetzten Nachkommenviren führen. Als Konsequenz wird die Infektionsausbreitung der Viren vermindert. Die Beobachtung, dass Proteasom-Inhibitoren den Lebenszyklus sowohl von Flaviviren als auch Pestiviren maßgeblich inhibieren, erlaubt den Rückschluss, dass diese Mittel in ähnlicher Weise auch auf die Infektion und auf die Infektionsausbreitung anderer Mitglieder der *Flaviviridae*-Familie wie Hepatitis-C-Viren und GB-Viren wirken, für die bisher keine infektiösen Zellkultursysteme etabliert werden konnten.

### Beispiel 2:

### Proteasomen-Inhibitoren haben nur geringe Effekte auf die intrazelluläre Replikation von BVDV- und HCV-RNA.

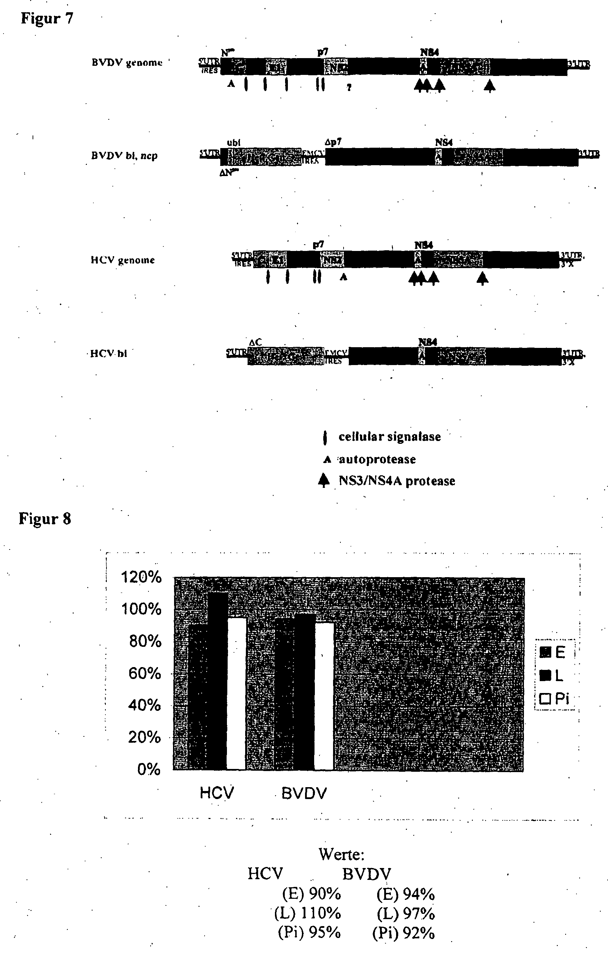

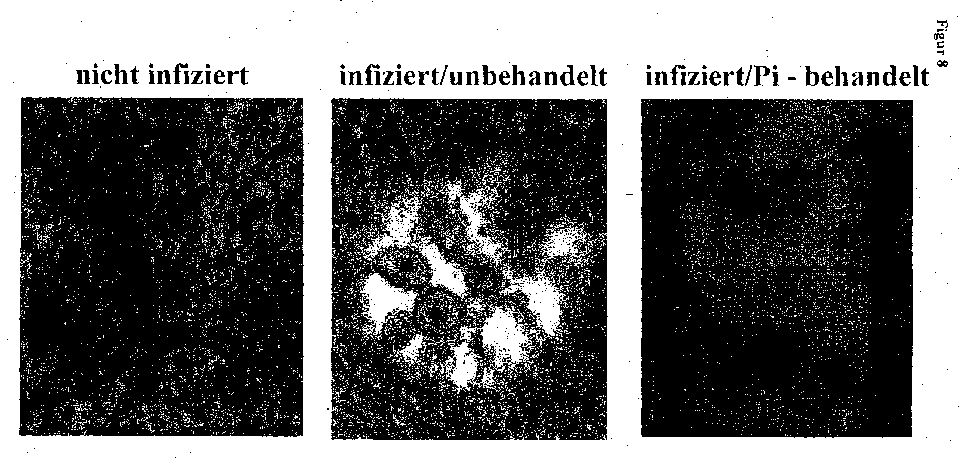

Zur eingehenderen Untersuchung des Effektes von Proteasom-Inhibitoren auf die intrazelluläre Replikation des viralen RNA-Genoms wurde auf Zelllinien zurückgegriffen, die ein persistentes BVDV-RNA-Replikon oder ein persistentes HCV-RNA-Replikon enthalten (Fig. 7 und 8). Für die Herstellung dieser Zelllinien (MDBK für BVDV, Huh-7 für HCV) wurden bizistronische RNA-Replikonkonstrukte verwendet, die neben dem für die viralen Proteine kodierenden ORF noch ein weiteres Gen enthalten, das für einen Selektionsmarker kodiert, also zum Beispiel das Gen für Hygromycin-B-Phosphotransferase. Hygromycin-B-Phosphotransferase inaktiviert das Antibiotikum Hygromycin B, einen Inhibitor der zellulären Translation. In den bizistronischen Konstrukten wird das heterologe Gen über die IRES der viralen 5'-UTR translatiert; die Expression der viralen Gene erfolgt über eine zusätzlich eingefügte IRES-Sequenz des Enzephalomyocarditis-Virus (EMCV). Im Falle von BVDV wurde für die Herstellung einer persistent transfizierten MDBK-Zelllinie naturgemäß ein nichtzytopathisches (nzp), NS2-3-kodierendes Replikon verwendet (siehe Einleitung und Fig. 7 und 8). Im Falle von HCV wurde eine NS3-exprimierende RNA zur Herstellung persistent transfizierter Huh-7-Zelllinien verwendet; diese induziert keinen ZPE (Lohmann *et al.,* 1999). Nach Transfektion der durch *in vitro*-Transkription gewonnenen RNAs (Standardprotokolle) wurden die Zellen unter selektionierenden Bedingungen, das heißt in der Gegenwart von 500 µg/ml Hygromycin, über mehrere Wochen gezüchtet. Da unter diesen Bedingungen nur Zellen überleben, die eine effiziente Hygromycin-Resistenz aufweisen, wurden so homogene Zelllinien selektioniert, die das entsprechende "Hyg-bici"-BVDV- oder "Hyg-bici"-HCV-Replikon persistent enthalten. Der Vorteil dieses Replikon-Zellsystems besteht vor allem darin, dass Effekte auf die RNA-Replikation unabhängig von Virus-Entry/Internalisierung, Uncoating bzw. der Assemblierung und Sekretion von Nachkommenviren untersucht werden können. Die Hyg-bici-BVDV oder Hyg-bici-HCV-Replikon-Zelllinien wurden für 24 bis 30 Stunden mit verschiedenen PI behandelt (siehe unten). Die Auswirkungen der Proteasom-Inhibitoren auf die intrazelluläre Replikation der viralen RNAs wurde wiederum durch Messung der Replikationsprodukte der viralen RNAs durch quantitative RNAse-Protektion bestimmt. Alternativ wurde der Effekt der Behandlung mit Proteasom-Inhibitoren auf die virale RNA-Replikation wie in Beispiel 1 beschrieben über Immunofluoreszenz-Tests oder durch Immunoblot-Verfahren (Standardprotokolle) verfolgt. Es wurde festgestellt, dass verschiedene PI keinen oder einen nur geringen Einfluss auf die intrazelluläre RNA-Replikation von BVDV- und HCV-RNA haben: In Fig. 7 und 8 ist gezeigt, dass die Replikationsrate der Hyg-bici-BVDV- oder Hyg-bici-HCV-Replikon-RNAs bei Behandlung mit Proteasom-Inhibitoren nur geringfügig (d.h. um max. 10%) sinkt.

Diese Ergebnisse zeigen, dass die Inhibition des zellulären Proteasoms keinen oder nur einen sehr geringen Einfluss auf die Translation der viralen RNA, auf die Prozessierung des viralen Nicht-Struktur Polyproteins und auf die Katalyse der beiden Replikationsschritte der viralen RNA hat. Aufgrund der Diskrepanz zu den Ergebnissen mit infizierten Zellen - wie in Beispiel 1 beschrieben, ließ sich in diesen Fällen ein klarer Effekt von PI auf die RNA-Replikation feststellen - konnte geschlossen werden, dass Proteasom-Inhibitoren offenbar Vorgänge wie Virus-Entry/Internalisierung und/oder -Uncoating inhibieren, die der RNA-Replikation vorangehen.

### Beispiel 3:

### Die Behandlung von Flaviviridae-infizierten Zellen mit Proteasom-Inhibitoren führt zu Unterschieden in der Anzahl der in infizierten Zellen detektierbaren Viruspartikel, zu Veränderungen des Verhältnisses kompletter zu nicht-kompletter Virionen sowie zu Veränderungen in der Morphologie sekretierter Nachkommenviren.

In den vorangegangenen Experimenten (Beispiel 1) wurde festgestellt, dass eine Inhibition der zellulären Proteasom-Aktivität die Freisetzung von infektiösen Nachkommenviren drastisch reduziert. Die gleichzeitige Feststellung, dass Proteasom-Inhibitoren weder direkt auf die Viruspartikel noch auf die Zellmembran wirken (Beispiel 1) und auch die RNA-Replikation nicht beeinflussen (Beispiel 2), führte zu der Hypothese, dass entweder der Entry/Internaliserungsvorgang bzw. das Uncoating oder aber die Assemblierung und die Sekretion von Nachkommenviren von der Inhibition des zellulären Proteasoms betroffen sein müssen. Zur Prüfung dieser Hypothese wurden MDBK-Zellen mit BVDV infiziert und in Gegenwart oder Abwesenheit von 10 µM MG 132 in speziellen Kapillaren mit einem Durchmesser von 200 µm für weitere 30 Stunden gezüchtet. Anschließend wurden die Zellen chemisch fixiert und für TEM prozessiert. Analog wurde mit Virusüberständen von mit Proteasom-Inhibitor behandelten infizierten Monolayerkulturen bzw. Mock-behandelten Kontrollen verfahren: Die entsprechenden Virus-haltigen Zellkultur-Überstände wurden in die Kapillaren transferiert, verschlossen, chemisch fixiert und für TEM präpariert. Bei der elektronenmikroskopischen Analyse wurde deutlich, dass sich in infizierten Zellen nach einer Behandlung mit PI eine signifikant geringere Anzahl an Viruspartikeln in zellulären Vesikeln im Vergleich zur Mock-Kontrolle detektieren lässt (Fig. 9). Zudem war in PI-behandelten Zellen das Verhältnis von komplett assemblierten zu nicht-komplett assemblierten Virionen stark zu ungunsten der komplett assemblierten Viruspartikel verschoben. Beim Vergleich der aus PI und Mock-behandelten Zellen gewonnenen virus-haltigen Überstände wurde deutlich, dass die Morphologie der unter PI-Behandlung sekretierten Viren im Vergleich zu Wild-Typ Viren deutlich verändert ist (Fig. 9). Diese Befunde stützen die Vorstellung, dass der inhibierende Effekt von Proteasom-Inhibitoren auf eine Infektion mit *Flaviviridae* den Assemblierungs und/oder den Sekretionsprozess der Nachkommenviren betrifft.

### Beispiel 4: Behandlung von Flaviviridae-infizierten Zellkulturen mit unterschiedlichen Klassen von Proteasom-Inhibitoren reduziert drastisch die Produktion von Nachkommenviren.

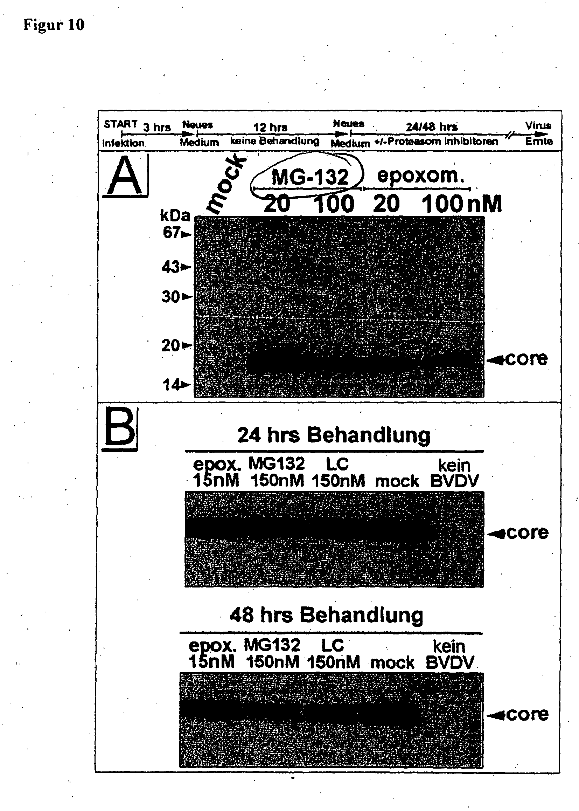

Parallelkulturen von MDBK-Zellen wurden wie in dem vorigen Beispiel beschrieben mit gleichen MOI des Pestivirus BVDV (CP7, NADL) infiziert. 3 Stunden nach Infektion wurden die Zellen geerntet, in PBS gewaschen und für weitere 12 Stunden kultiviert. Durch diesen ersten Waschschritt wurde das Input-Virus komplett aus der Kultur entfernt, und es können daher nur neu produzierte Nachkommenviren nachgewiesen werden. Nach weiteren 12 Stunden Behandlung wurden die Zellen ebenfalls geerntet, gewaschen, in gleichen Zellmengen aliquotiert und mit verschiedenen Proteasom-Inhibitoren für 24 oder 48 Stunden behandelt. Nach dieser Behandlungszeit wurden die Viren durch Zentrifugation aus identischen Volumen von Zellkulturüberständen durch Zentrifugation (99 min, 20.000xg, 4°C) gewonnen. Der jeweilige Zeitablauf für Infektion, Behandlung und Virusemte ist in Figur 10 dargestellt. Die pelletierten Virionen wurden in SDS-Probenpuffer denaturiert und im Western-Blot mit anti-BVDV-core-Antikörpern analysiert. In Figur 10, Teil A wurden die Western-Blots mit Antikörpern angefärbt, welche das reife und prozessierte BVDV-core erkennen; in Figur 10, Teil B mit Antikörpern, welche auch das unreife nicht prozessierte core-Protein erkennen. Dabei ist deutlich zu sehen, dass eine klare Abnahme der Menge an BVDV-core-Protein auftritt, welche von der Dosis der Proteasom-Inhibitoren abhängt. Der Effekt von 100 nM Epoxomicin war etwa vergleichbar mit dem inhibitorischen Effekt von 200nM des Peptidaldehyds MG132. Die Hemmung der Produktion von BVDV wurde weiterhin verstärkt wenn die Behandlungsdauer auf 48 Stunden verlängert wurde (Figur 10, Teil B). Hierzu wurden Zellen mit den Proteasom-Inhibitoren Epoxomicin, MG132 sowie mit dem beta-Lacton Lactacystin behandelt (Figur 10, Teil B). Dabei zeigte sich eine Dosis-abhängige Verringerung des core-Signals, welches sowohl die reife als auch die unreife Form des BVDV-core-Proteins betraf. Gemessen am core-Signal ist nach 48 Stunden Behandlung mit 150 nM von MG132 oder Lactacystein eine Abnahme der Menge von BVDV Virus auf 10 Prozent zu verzeichnen (Figur 10, Teil B). In Übereinstimmung mit den vorherigen Beispielen wird mit diesem Beispiel weiterführend gezeigt, dass chemisch unterschiedliche Klassen von Proteasom-Inhibitoren die Produktion von BVDV-Virionen blockieren. Dies konnte anhand der biochemischen Analyse von pelletierten Virionen direkt nachgewiesen werden. Da dieser Effekt für Proteasom-Inhibitoren der Klasse von Peptidealdehyden (MG 132), Epoxyketonen (Epoxymicin), Peptidvinylsulfonen (NLVS, Daten nicht gezeigt), und Lactonen (LC) nachgewiesen wurde, kann somit davon ausgegangen werden, dass das 26S Proteasom für die Produktion von BVDV und damit anderen Vertretern der *Flaviviridae* notwendig ist, beziehungsweise Proteasom-Inhibitoren die Replikation von *Flaviviridae* blockieren können.

### Legende zu den Figuren

### Figur 1: Effekt von PI auf eine Flaviviridae-Infektion

### Teil 1: Effekt einer Vorbehandlung von Zellen mit PI auf eine anschließende Infektion mit Flaviviridae.

BHK-21- bzw. MDBK-Zellen wurden für 20 Stunden bei einer Konzentration von 10nM Epoxomycin (E), Lactacystin (L) bzw. dem Proteasom-Inhibitor (Pi) MG132 im Kulturmedium kultiviert. Nach Waschen der Zellen mit PBS wurden diese mit West-Nil-Virus (BHK-21-Zellen) bzw. BVDV CP7 (MDBK-Zellen) bei einer MOI von 1-5 infiziert. 12 bzw. 30 Stunden nach der Infektion wurden der Titer und die spezifische Infektiosität der im Zellkultur-Überstand befindlichen Nachkommenviren (Protokolle siehe Anwendungsbeispiel 1) ermittelt.

Angegeben ist der Titer infektiöser West-Nil-Virus und BVDV-Nachkommenviren, die nach Infektion von PI-vorbehandelten im Vergleich zu Mock-vorbehandelten MDBK- bzw. BHK-Zellen freigesetzt wurden (Angaben als Verhältnis PI-vorbehandelt/Mockvorbehandeln in %; Mock-vorbehandelt = 100%).

Angegeben ist weiterhin die spezifische Infektiosität der Nachkommenviren (Angaben wiederum als Verhältnis PI-vorbehandelt/Mock-vorbehandelt in %; Mock-vorbehandelt = 100%). Die Darstellung entspricht Mittelwerten aus drei unabhängigen Experimenten (Standardabweichung ca. 10%).

### Fig. 2: Effekt von PI auf eine Flaviviridae-Infektion

### Teil 2: Konsequenzen einer Vorbehandlung von West-Nil-Viren und BVDV-CP7-Viren mit PI auf deren Infektiosität.

West-Nil-Virus-haltige bzw. BVDV-haltige Kulturüberstände wurden von infizierten Zellen gewonnen und der Virustiter bestimmt (Protokolle siehe Anwendungsbeispiel 1). 1ml der Überstände wurden je auf eine Konzentration von 10 nM verschiedener PI (Epoxomycin, Lactacystin, MG132) eingestellt. Die mit PI versetzten Überstände und eine entsprechende Mock-Kontrolle wurden für 30 Stunden bei 37° (Zellkulturbedingungen) inkubiert und anschließend zur Infektion von BHK-Zellen (West-Nil-Virus) und MDBK-Zellen (BVDV) eingesetzt. Der Titer und die spezifische Infektiosität der resultierenden Nachkommenviren wurden mit den unter Anwendungsbeispiel 1 beschriebenen Protokollen ermittelt.

Angegeben ist der Titer infektiöser BVDV- und West-Nil-Virus-Nachkommenviren, freigesetzt aus MDBK- bzw. BHK-Zellen, die zuvor entweder mit PI-behandelten oder Mock-behandelten Virus-Überständen infiziert wurden (Angaben als Verhältnis PIvorbehandelt/Mock-vorbehandelt in %; Mock-vorbehandelt = 100%). Angegeben ist weiterhin die spezifische Infektiosität der Nachkommenviren (Angaben wiederum als Verhältnis PIvorbehandelt/Mock-vorbehandelt in %; Mock-vorbehandelt = 100%). Die Darstellung entspricht Mittelwerten aus drei unabhängigen Experimenten (Standardabweichung ca. 10%).

### Fig. 3-5: Effekt von PI auf eine Flaviviridae-Infektion

### Teil 3: Effekt einer PI-Behandlung auf bereits infizierte Zellen.

BHK-21- und MDBK-Zellen wurden mit BVDV bzw. West-Nil-Virus infiziert (Protokolle siehe Anwendungsbeispiel 1) und das Kulturmedium direkt im Anschluss an die Infektion auf 10nM verschiedener PI (Epoxomycin (E), Lactacystin (L), MG132 (Pi)) eingestellt. Nach Einstellung eines ZPE, i.d.R. 12 Stunden (West-Nil-Virus) bzw. 30 Stunden (BVDV) nach der Infektion, wurde der Titer und die spezifische Infektiosität der freigesetzten Nachkommenviren ermittelt. Darüber hinaus wurde die Replikationsrate der viralen RNAs in den infizierten Zellen bestimmt (Protokolle erläutert in Anwendungsbeispiel 1).

Titer infektiöser West-Nil- und BVDV-Nachkommenviren, freigesetzt aus BHK- bzw. MDBK-Zellen, die nach der Infektion mit der angegebenen Konzentration und für die angegebenen Zeiten mit verschiedenen Proteasom-Inhibitoren (siehe Fig. 1 und 2) behandelt wurden. Angegeben sind die Mittelwerte aus drei unabhängigen Virus-Titrationen mit jeweils unabhängigen Mock-Kontrollen (Standardabweichung ca. 20%).

Spezifische Infektiosität der Nachkommenviren im Verhältnis zu den mock-behandelten Kontrollen angegeben als Verhältnis PI-behandelt/Mock-behandelt in % (Mock-behandelt = 100%). Angegeben sind die Mittelwerte aus drei unabhängigen Experimenten (Standardabweichung ca. 20%).

Replikationsrate der Viren, ermittelt über RNase-Protektion der neu synthetisierten Positiv-Strang-RNA (Grassmann *et al.,* 1999) angegeben als Verhältnis PI-behandelt/Mock-behandelt in % (Mock-behandelt = 100%). Angegeben sind die Mittelwerte aus drei unabhängigen Experimenten (Standardabweichung ca. 10%).

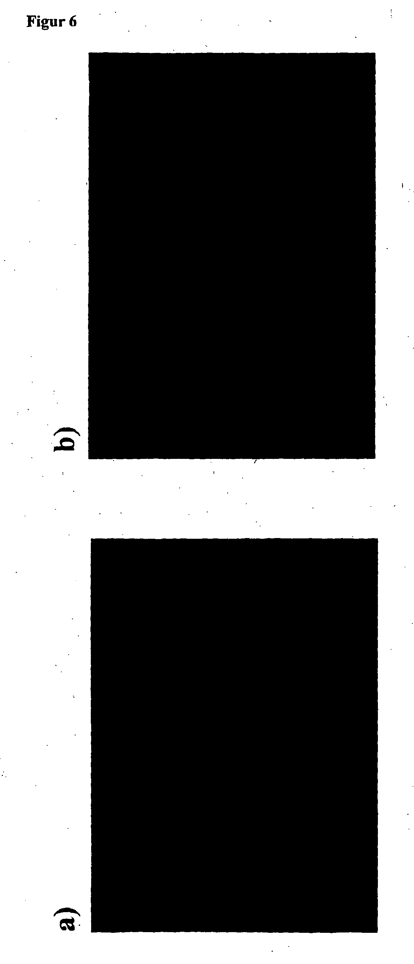

### Fig. 6: Effekt einer PI-Behandlung Flaviviridae-infizierter Zellen auf die Ausbreitung der Infektion in Zellkultur.

MDBK-Zellen wurden entsprechend den im Detail in Ausführungsbeispiel 1 erläuterten Protokollen mit BVDV CP7 infiziert und mit dem Proteasom-Inhibitor Pi behandelt. 30 Stunden nach der Infektion wurden die Proteasom-Inhibitor-behandelten Zellen und, zum Vergleich, unbehandelte und sonst unter identischen Bedingungen infizierte Zellen einem Immunfluoreszenztest mit anti-BVDV-NS3-Antikörper unterzogen (Behrens *et al.,* 1998). Gezeigt sind die durch Immunfluoreszenz des viralen NS3-Proteins sichtbar gemachten Zellen (100-fach mikroskopisch vergrößert) in a) Abwesenheit des Proteasom-Inhibitors und b) nach Behandlung mit 10 nM Proteasom-Inhibitor MG132 (Pi).

### Fig. 7 und 8: Effekt einer PI-Behandlung auf die Replikationsfähigkeit von BVDV- und HCV-Replikon-RNA

Organisation bizistronischer BVDV- und HCV-RNA-Replikons im Vergleich zur Organisation des BVDV- und HCV-Gesamtgenoms. Waagerechte Linien kennzeichnen die nicht-translatierten Regionen (UTRs); Boxen symbolisieren die translatierten Bereiche der viralen RNAs. Die Art der proteolytischen Aktivität, die an den gekennzeichneten Stellen des Polyproteins zur Spaltung führt, ist durch ein entsprechendes Symbol unter der Spaltstelle angegeben. "Het. Gen" symbolisiert das in den bi-zistronischen RNAs exprimierte zusätzliche Gen (z.B. Hygromycin-B-Phosphatransferase). Aus Gründen der Prozessierung des N-Terminus von NS2-3 enthält das nzp BVDV-Replikon noch einen Teil des p7-kodierenden Bereiches. Ein Teilbereich des N^{pro}-Gens (N-terminale Autoprotease von BVDV) und ein Teilbereich des Capsid-Gens (bei HCV) dient der besseren Effizienz der IRES-mediierten Translation des Polyproteins Zur Generierung des authentischen N-Terminus der Proteine wurde z.T. ein Ubiquitingen ("Ubi") eingefügt: Auf diese Weise erfolgt die proteolytische Prozessierung des Polyproteins an dieser Stelle über die zelluläre Ubiquitin carboxy-terminale Hydrolase (Behrens *et al.,* 1998; für Review, siehe Lindenbach and Rice, 2001; weitere Erläuterungen im Text).

Homogene MDBK- bzw. Huh-7-Zelllinien, die das BVDV- bzw. HCV-Replikon persistent enthielten, wurden über mindestens 30 Stunden mit den verschiedenen Proteasom-Inhibitoren Epoxomycin (E), Lactacystin (L) und dem Proteasom-Inhibitor MG 132 (Pi) (behandelt. Im Anschluss daran wurden die Zellen von der Kulturplatte gelöst und die zytoplasmatische RNA über die in den Anwendungsbeispielen 1 und 2 beschriebenen Verfahren extrahiert. Der Nachweis und die Quantifizierung neu synthetisierter viraler Positiv-Strang-RNA erfolgte durch RNase-Protektion. In der Abbildung wird die Quantität der aus PI-behandelten Zellen extrahierten viralen RNA mit der aus Mock-behandelten Zellen extrahierten viralen RNA verglichen: Angegeben ist das Verhältnis der aus PI-behandelten Zellen gewonnenen Quantität an viraler RNA zu der aus Mock-behandelten Zellen gewonnenen Quantität an viraler RNA in % (Mock-behandelte RNA = 100%). Angegeben sind die Mittelwerte aus drei unabhängigen Experimenten (Standardabweichung ca. 10%). Immunofluoreszenztests ergaben konforme Ergebnisse (nicht gezeigt).

### Fig. 9: Elektronenmikroskopische Studien PI-behandelter und Mock-behandelter MDBK-Zellen nach Infektion mit BVDV

MDBK-Zellen wurden entsprechend dem unter Anwendungsbeispiel 3 erläuterten Protokoll mit BVDV CP7 infiziert und elektronenmikroskopisch analysiert.

### Fig. 10:

Unterschiedliche Klassen von PI blockieren die Produktion von BVDV CP7.

MDBK-Zellen wurden entsprechend den vorherigen Anwendungsbeispielen mit BVDV (CP7, NADL) infiziert. Zur vollständigen Entfernung des Inputvirus wurden die Zellen gewaschen und anschließend für 12 Stunden weiter behandelt, um eine vollständige Etablierung der Infektion zu ermöglichen. Nach erneuter Waschung wurden die Zellen für 24 oder 48 Stunden mit unterschiedlichen Konzentrationen der Proteasom-Inhbitoren MG 132, Epoxomycin, oder Lactacystin (LC) behandelt. Die freigesetzten Viren wurden geerntet und im Western-blot mit anti-BVDV-core-Antikörpern analysiert, welche entweder nur reifes (A) oder auch nicht prozessiertes core-(B)-Protein erkennen.

### Abkürzungsverzeichnis

- ATP: Adenosin-5'-triphosphat
- BHK: Baby Hamster Kidney
- BVDV: Virus der bovinen viralen Diarrhoe
- cDNA: copy DNA
- CFTR: Cystis Fibrosis Transmembran Regulator
- CSFV: Virus der klassischen Schweinepest
- Da: Dalton (Maß für Molekulargewicht)
- DHBV: Enten-Hepatitis-B-Virus
- DNA: desoxyribonucleic acid (Desoxyribonukleinsäure)
- DTT: Dithiothreitol
- EDTA: Ethylendiamintetraessigsäure
- EM: Elektronenmikroskopie
- EMCV: Enzephalomyocarditis-Virus
- FDA: Fluoresceindiazetat
- FSME: Frühsommer-Meningoenzephalitis
- GB-Viren: Hepatitis-GB-Viren
- HAART: highly active antiretroviral therapy
- HAART-Therapie: hoch aktive antiretrovirale Therapie
- HAV: Hepatitis-A-Virus
- HBV: Hepatitis-B-Virus
- HCC: Hepatozelluläres Karzinom
- HCV: Hepatitis-C-Virus
- HDV: Hepatitis-Delta-Virus
- HEPES: N-[2-hydroxyethyl]piperazine-N'-[2-ethanesulfonic acid]
- HEV: Hepatitis-E-Virus
- HFV: Hepatitis-F-Virus
- HGV: Hepatitis-G-Virus
- HIV: Humanes Immundefizienzvirus
- HPV: Humanes Papilloma-Virus
- Huh-7: humane transformierte Hepatomazellen
- i.d.R.: in der Regel
- IFN: Interferon
- IL: Interleukin
- IRES: interne Ribosomen-Eintrittsstelle
- kb: Kilobasen
- IκB: inhibitorischer Faktor IκB
- kDa: Kilodalton (Maß für Molekulargewicht)
- Ki: inhibitorische Konstante
- LC: Lactacystin
- LDL: Low density lipoprotein
- MDa: Megadalton
- MDBK: Marbin Darby Bovine Kindey
- MHC: Major Histocompatibilitäts Komplex
- µM: micromo lar
- MG 132: Proteasom-Inhibitor "MG 132"
- mM: millimo lar
- MOI: multiplicity of infection = Anzahl der pro Zelle eingesetzten infektösen Viruspartikel
- NF-κB: Transkriptionsfaktor
- NLVS: 4-Hydroxy-5-iodo-3-nitrophenylactetyl-L-Leucinyl-L-Leucinyl- L-Leucin-vinyl-sulfon
- nM: nanomolar
- nzp: nicht zytopathisch
- ORF: open reading frame (translationales Leseraster)
- PBS: Phosphatpuffer, phosphate buffered saline
- PCR: polymerase chain reaction - Polymerase-Kettenreaktion
- PGPH: Postglutamyl-Peptid hydrolysierende
- PNGase: Peptid N-Glykosidase
- PI: Proteasom-Inhibitor(en)
- PK: Proteinase K
- PS: ProScript
- PS-341: N-Pyrazinecarbonyl-L-Phenylalanin-L-leuzin-Borsäure
- PS-519: 1*R*-[1*S*,4*R*,5*S*]]-1-(1-Hydroxy-2-methylpropyl)-4-propyl-6- oxa-2-azabicyclo[3.2.0]heptane-3,7-dion
- PS-273: Morpholin-CONH-(CH-Naphthyl)-CONH-(CH-isobutyl)-B(OH)₂
- PSI: N-Carbobenzoxy-Ile-Glu(OBut)-Ala-Leu-H
- RdRp: RNA-abhängige RNA-Polymerase
- RNA: Ribonukleinsäure (ribonucleic acid)
- RNase: Ribonuklease
- RT: Reverse Transkriptase
- SDS: Natriumdodecylsulfat (Sodium Dodecylsulfate)
- SDS-PAGE: SDS-Polyacrylamid Gelelektrophorese
- TEM: Transmissions-Elektronenmikroskopie
- TNF: Tumornekrosefaktor
- Tris: Tris-Puffer - Tris-(hydroxymethyl)-aminomethan
- Ub: Ubiquitin
- UCH: Ubiquitincarboxyhydrolase
- UPS: Ub-Proteasom-System
- UTR: nicht-translatierte Region(en) (UTRs)
- zLLL: N-carbobenzoxyl-L-leucinyl-L-leucinyl-L-leucinal
- zp: zytopathisch
- ZPE: zytopathischer Effekt der Virusinfektion

### Literaturverzeichnis

Adams, J; Behnke, M; Chen, S; Cruickshank, AA; Dick, RL; Grenier, L; Klunder, JM; Ma, Y.-T; Plamondon, L; Stein, RL (1998). Potent and selective inhibition of the proteasome: dipeptidyl boronic acids. Bioorg. Med. Chem. Lett. 8:333-338.
Adams, J; Palombella, VJ; Sausville, EA; Johnson, J; Destree, A; Lazarus, DD; Maas, J; Pien, CS; Prakash, S; Elliott, PJ (1999). Proteasome inhibitors: a novel class of potent and effective antitumor agents. Cancer Res. 59:2615-2622.
Adams, J; Palombella, VJ; Elliot, PJ (2000). Proteasome inhibition: a new strategy in cancer treatment. Investigational New drugs 18:109-121.
Adams, J; Stein, R (1996). Novel inhibitors of the proteasome and their therapeutic use in inflammation. Annu. Rep. Med. Schein. 31:279-288.
Agnello, V, Abel, G, Elfahal, M, Knight, GB, Zhang, QX; (1999). Hepatitis C virus and other flaviviridae viruses enter cells via low density lipoprotein receptor. Proc Natl Acad Sci U S A. 96:12766-71.
Agnello, V; (2000). Mixed cryoglobulinemia amd other extrahepatic manifestations of hepatitis C virus infections. In: Liang, TJ, Hoonagle JH, eds. Hepatitis C. San Diego: Academic Press: 295-314.
Baldwin, AS (1996). The NF-kappa B and I kappa B proteins: new discoveries and insights. Annu. Rev. Immunol. 14:649-683.
Barton, DJ, Flanegan JB; (1993). Coupled translation and replication of poliovirus RNA in vitro: synthesis of functional 3D polymerase and infectious virus. J. Virol. 67: 822-831. Behrens, SE, Grassmann, CW, Thiel, HJ, Meyers, G, Tautz, N; (1998). Characterization of an autonomous subgenomic RNA-replicon of a pestivirus. J. Virol. 72: 2364-2372.
Bukh, J, Miller RH, Purcell, RH; (1995). Genetic heterohgeneity of hepatitis C virus: quasispecies and genotypes. Sem.Liver Dis. 15: 41-63.
Burke, DS, Monath, TP; (2001). Flaviviruses. In: Virology, fourth edition edited by B.N. Fields, Lippincott-Raven Philadelphia, New York: 1043=1125.
Ciechanover, A (1998). The ubiquitin-proteasome pathway: on protein death and cell live. EMBO J. 17: 7151-7160.
Ciechanover, A, Orian, A, Schwartz, AL; (2000).The ubiquitin-mediated proteolytic pathway: mode of action and clinical implications. J. Cell Biochem. Suppl. 34: 40-51.
Coux, O; Tanaka, K; Goldberg, AL (1996). Structure and functions of the 20S and 26S proteasomes. Annu. Rev. Biochem. 65: 801-847.
Dubuisson, J, Hsu, HH, Cheung, RC, Greenberg, HB, Russell, DG, Rice, CM; (1994). Formation and intracellular localization of hepatitis C virus envelope glycoprotein complexes expressed by recombinant vaccinia and Sindbis virus. J. Virol. 68: 6147-6160.
Elliott, PJ, Ross JS; (2001).The proteasome: a new target for novel drug therapies. Am J Clin Pathol. 116: 637-46.
Elliott, PJ; Pien, CS; McCormack, TA; Capman, ID; Adams, J (1999). Proteasome inhibition: a novel mechanism to combat asthma. J. Allergy Clin. Immunol. 104: 294-300.
Fenteany, G; Standaert, RF; Lane, WS; Choi, S; Corey, EJ; Schreiber, SL (1995). Inhibition of proteasome activities and subunit-specific amino-terminal threonine modification by lactacystein. Science 268: 726-731.
Frankel, A; Man, S; Elliott, P; Adams, J; Kerbel, RS (2000). Lack of multicellular drug resistance observed in human ovarian and prostate carcinoma treated with the proteasome inhibitor PS-341. Clinical Cancer Res. 6:3719-3728.
Fuerst, TR, Niles, EG, Studier, FW, Moss B; (1986). Eukaryotic transient-expression system based on recombinant vaccinia virus that synthesizes bacteriophage T7 RNA polymerase. Proc.Natl.Acad.Sci. USA 83: 8122-8126.
Grassmann, CW, Isken, O, Behrens SE; (1999). Assignment of the multifunctional NS3 protein of bovine viral diarrhea virus during RNA replication: an in vivo and in vitro study. J. Virol. 73: 9196-9205.
Grassmann, CW, Isken, O, Tautz, N, Behrens SE; (2001). Genetic analysis of the pestivirus nonstructural coding region: defects in the NS5A unit can be complemented in trans. J. Virol.75: 7791-7802.
Hanada, M; Sugawara, K; Kaneta, K; Toda, S; Nishiyama, Y; Tomita, K; Yamamoto, H; Konishi, M; Oki, T (1992). Epoxomicin, a new antitumor agent of microbial origin. J. Antibiot. (Tokyo) 45:1746-1752.
Harty, RN; Brown, ME; Wang, G; Huibregtse, J; Hayes, FP; (2000). A PPxY motif within the VP40 protein of Ebola virus interacts physically and functionally with a ubiquitin ligase: implications for filovirus budding. Proc. Natl. Acad. Sci. USA 97: 13871-13876.
Hershko, A; Ciechanover, A; (1998). The ubiquitin system. Annu. Rev Biochem. 67: 425-479. Higashitsuji,H; Itoh, K; Nagao, T; Dawson, S; Nonoguchi, K; Kido, T; Mayer, RJ; Arii, S; Fujita, J; (2000). Reduced stability of retinoblastoma protein by gankyrin, an oncogenic ankyrin-repeat protein overexpressed in hepatomas. Nat Med. 6:96-99.
Köck J, Nassal M, MacNelly S, Baumert TF, Blum HE, von Weizsäcker F; (2001). Efficient infection of primary Tupaia hepatocytes with purified human and woolly monkey hepatitis B virus. J. Virol. 75:5084-5089.
Lohmann, V, Körner, F, Koch, JO, Herian, U, Theilmann, L., Bartenschlager R; (1999). Science 285:110-113.
Lightcap, ES; McCormack, TA; Pien, CS; Chau, V; Adams, J; Elliott, PJ (2000). Proteasome inhibition measurements: clinical application. Clin. Chem. 46:673-683.
Lindenbach, BD, Rice, CM; (2001). Flaviviridae: the viruses and their replication. In: Virology, fourth edition edited by B.N. Fields, Lippincott-Raven Philadelphia, New York: 991-1042.
Major, ME, Rehermann, B, Feinstone, SM; (2001). Hepatitis C viruses. In: Virology, fourth edition edited by B.N. Fields, Lippincott-Raven Philadelphia, New York: 1127-1161.
Meng, L; Kwok, BH; Sin, N; Crews, CM (1999a). Eponemycin exerts its antitumor effect through the inhibition of proteasome function. Cancer Res. 59:2798-2801.
Meng, L; Mohan, R; Kwok, BH; Elofsson, M; Sin, N; Crews, CM (1999b). Epoxomicin, a potent and selective proteasome inhibitor, exhibits in vivo antiinflammatory activity. Proc. Natl. Acad. Sci. USA. 96(18): 10403-10408.
Merola, M, Brazzoli, M, Cochiarella, F, Heile, JM, Helenius, A, Weiner, AJ, Houghton, M, Abrignani, S; (2001). Folding of Hepatitis C Virus E1 glycoprotein in a cell-free system. J. Virol. 75: 11205-11217.
Meyers, G, Tautz, N, Becher, P, Thiel, H-J, Kümmerer BM; (1996). Recovery of cytopathogenic and noncytopathogenic bovine viral diarrhea viruses from cDNA constructs. J. Virol. 70: 8606-8613.
Moormann, RJM, van Gennip, HGP, Miedema, GKL, Hulst, MM, van Rijn PA; (1996). Infectious RNA transcribed from an engineered full-length cDNA template of the genome of a pestivirus. J. Virol. 70: 763-770.
Moradpour, D; Grabscheid, B; Kammer, AR; Schmidtke, G; Gröttrup, M; Blum, HE; Cerny, A (2001). Expression of hepatitis C virus proteins does not interfere with major histocompatibility complex class I processing and presentation in vitro. Hepatology 33:1282-1287.
Müller, K, Heimann, M, Rümenapf, T; (2000). Evidences that CD46 is involved in BVDV infection. Keystone symposium: Cell biology of virus entry, replication and pathogenesis. Taos, NM, Abstract supplement.
Palombella, VJ; Conner, EM; Fuseler, JW; Destree, A; Davis, JM; Laroux, FS; Wolf, RE; Huang, J; Brand, S; Elliott, PJ; Lazarus, D; McCormack, T; Parent, L; Stein, R; Adams, J; Grisham, MB; (1998). Role of the proteasome and NF-kappaB in streptococcal cell wallinduced polyarthritis. Proc. Natl. Acad. Sci. USA 95:15671-15676.
Palombella, VJ; Rando, OJ; Goldberg, AL; Maniatis, T; (1994). The ubiquitin-proteasome pathway is required for processing the NF-kappa B1 precursor protein and the activation of NF-kappa B. Cell 78:773-785.
Pamer, E; Cresswell, P; (1998). Mechanisms of MHC class I-restricted antigen processing. Annu. Rev. Immunol. 16:323-358.
Patnaik, A, Chau, V, Wills, JW; (2000). Ubiquitin is part of the retrovirus budding machinery. Proc. Natl. Acad. Sci. USA 97:13069-13074.
Pietschmann, T, Lohmann, V, Kaul, A, Krieger, N, Rinck, G, Rutter, G, Strand, D, Bartenschlager, R; (2002) Persistent and transient replication of full-length hepatitis C virus genomes in cell culture. J. Virol. 76:4008-21.
Phillips, JB; Williams, AJ; Adams, J; Elliott, PJ; Tortella, FC (2000). Proteasome inhibitor PS-519 reduces infarction and attenuates leukocyte infiltration in a rat model of focal cerebral ischemia. Stroke 31:1686-1693.
Rehermann, B; Ferrari, C; Pasquinelli, C; Chisari, FV (1996). The hepatitis B virus persists for decades after patients' recovery from acute viral hepatitis despite active maintenance of a cytotoxic T-lymphocyte response. Nat Med. 2:1104-1108.
Rock, KL; Gramm, C; Rothstein, L; Clark, K; Stein, R; Dick, L; Hwang, D; Goldberg, AL (1994). Inhibitors of the proteasome block the degradation of most cell proteins and the generation of peptides presented on MHC class I molecules. Cell 78:761-771.
Rock, KL; Goldberg, AL (1999). Degradation of cell proteins and the generation of MHC class I-presented peptides. Annu. Rev. Immunol. 17:739-779.
Rümenapf, T., Unger, G, Strauss, JH, and Thiel, HJ; (1993). Processing of the envelope glycoproteins of pestiviruses. J.Virol. 67: 3288-3294.
Schubert, U; Antön, LC; Gibbs, J; Norbury, CC; Yewdell, JW; Bennink, JR (2000b). Rapid degradation of a large fraction of newly synthesized proteins by proteasomes. Nature 404:770-774.
Schubert, U; Ott, DE; Chertova, EN; Welker, R; Tessmer, U; Princiotta, MF; Bennink, JR; Kräusslich, H-G; Yewdell, JW (2000a). Proteasome inhibition interferes with gag polyprotein processing, release, and maturation of HIV-1 and HIV-2. Proc. Natl. Acad. Sci. USA 97:13057-13062.
Schwartz, AL; Ciechanover, A (1999). The ubiquitin-proteasome pathway and pathogenesis of human diseases. Annu. Rev. Med. 50:57-74.
Strack, B; Calistri, A; Accola, MA; Palu, G; Göttlinger, HG (2000). A role for ubiquitin ligase recruitment in retrovirus release. Proc. Natl. Acad. Sci. USA 97:13063-13068.
Suzuki, R; Tamura, K; Li, J; Ishii, K; Matsuura, Y; Miyamura, T; Suzuki, T (2001). Ubiquitin-mediated degradation of hepatitis C virus core protein is regulated by processing at its carboxyl terminus. Virology 280:301-309.
Teicher, BA; Ara, G; Herbst, R; Palombella, VJ; Adams, J (1999). The proteasome inhibitor PS-341 in cancer therapy. Clin. Cancer Res. 5:2638-2645.
Thiel, HJ, Plagemann, PGW, Moennig,V; (1996). Pestiviruses. In B.N. Fields, D.M.Knipe, and P.M. Howley (ed.), Fields virology. Raven Press, Philadelphia. Pa.: 1059-1074. Trautwein, C; Manns, M (2001). Antivirale Therapie der chronischen Virushepatitis. Internist 42:913-923.
Yagi, T; Hardin, JA; Valenzuela, YM; Miyoshi, H; Gores, GJ; Nyberg, SL (2001). Caspase inhibition reduces apoptotic death of cryopreserved porcine hepatocytes. Hepatology 33:1432-40.
Yu, H, Grassmann, CW, Behrens SE; (1999). Sequence and structural elements at the 3' terminus of bovine viral diarrhea virus genomic RNA: functional role during RNA replication. J. Virol. 73: 3638-3648.
Yu, H, Isken, O, Grassmann, CW, Behrens SE; (2000). A stem-loop motif formed by the immediate 5'-terminus of the bovine viral diarrhea virus genome modulates translation as well as replication of the viral RNA. J. Virol. 74: 5825-5835.
Zhao, X, Tang Z, Klumpp B, Wolff-Vorbeck, G, Barth H, Levy S, von Weizsäcker, F, Blum HE, Baumert TF; (2002). Primary hepatocytes of Tupaia belangeri as a potential model for hepatitis C virus infection J. Clin. Invest. 109: 221-232.

## Patentansprüche

1. Proteasom-Inhibitoren zur Anwendung in einem Verfahren zur Behandlung von Erkrankungen, die durch die Genera Flavivirus oder Pestivirus der Familie Flaviviridae verursacht werden.

2. Proteasom-Inhibitoren nach Anspruch 1 zur Behandlung und Prophylaxe von Flavivirus-bedingtem Fieber, Hämorrhagien, Leukopenie, Thrombozytopenie, Durchfallerkrankungen und Encephalitiden sowie Pestivirus-verursachten Krankheiten.

3. Proteasom-Inhibitoren nach Anspruch 1 oder 2, **dadurch gekennzeichnet, dass** Substanzen eingesetzt werden,
3.1. welche die Aktivitäten des Ubiquitin/Proteasom-Pathway hemmen, regulieren oder anderweitig beeinflussen
3.2. die speziell die enzymatischen Aktivitäten des kompletten 26S Proteasom-Komplexes und
3.3. die speziell die enzymatischen Aktivitäten des freien, nicht mit regulatorischen Untereinheiten assemblierten 20S katalytisch aktiven Proteasom-Komplexes beeinflussen.

4. Proteasom-Inhibitoren nach einem der Ansprüche 1 bis 3, **dadurch gekennzeichnet, dass** Substanzen eingesetzt werden, die als Proteasom-Inhibitoren von Zellen höherer Eukaryoten aufgenommen werden und nach Zellaufnahme mit den katalytischen Untereinheiten des Proteasoms in Wechselwirkung treten und dabei alle oder einzelne proteolytische Aktivitäten des Proteasoms - die Trypsin-, die Chymotrypsin- und die Postglutamyl-Peptid hydrolysierenden Aktivitäten - innerhalb des 26S oder auch des 20S Proteasomkomplexes blockieren.

5. Proteasom-Inhibitoren nach einem der Ansprüche 1 bis 4, **dadurch gekennzeichnet, dass** sie zusammen mit anderen Substanzen eingesetzt werden, die das zelluläre Ubiquitin-System beeinflussen, regulieren oder hemmen, wie die Aktivitäten
5.1. der Ubiquitin-konjugierenden Enzyme und/oder
5.2. der Ubiquitin-hydrolysierenden Enzyme.

6. Proteasom-Inhibitoren einem der Ansprüche 1 bis 5, **dadurch gekennzeichnet, dass** Substanzen eingesetzt werden, die in verschiedenen Formen *in vivo* oral, intravenös, intramuskulär, subkutan oder in verkapselter Form mit oder ohne Zell-Spezifität-tragende Veränderungen verabreicht werden können, aufgrund der Anwendung eines bestimmtes Applikations- und/oder Dosis-Regimes eine geringe Zytotoxizität aufweisen, keine oder unbedeutende Nebenwirkungen auslösen, eine relative hohe metabolische Halbwertszeit und eine relative geringe Clearance-Rate im Organismus aufweisen.

7. Proteasom-Inhibitoren einem der Ansprüche 1 bis 6, **dadurch gekennzeichnet, dass** Substanzen eingesetzt werden, die
a) in natürlicher Form aus Mikroorganismen oder anderen natürlichen Quellen isoliert werden oder
b) durch chemische Modifikationen aus natürlichen Substanzen hervorgehen oder
c) total-synthetisch hergestellt werden oder
d) durch gentherapeutische Verfahren *in vivo* synthetisiert werden.

8. Proteasom-Inhibitoren einem der Ansprüche 1 bis 7, **dadurch gekennzeichnet, dass** Substanzen eingesetzt werden, die folgenden Substanzklassen angehören:
8.a) natürlich vorkommende Proteasom-Inhibitoren:
- Peptid-Derivate, welche C-terminal Epoxyketon-Strukturen enthalten
- β-Lacton-Derivate
- Aclacinomycin A (auch bezeichnet als Aclarubicin)
- Lactacystin und dessen chemische modifizierte Varianten, wie der Zellmembranpenetrierenden Variante "Clastolactacystein β-Lacton"
8.b) synthetisch hergestellte Proteasom-Inhibitoren:
- modifizierte Peptidaldehyde, wie N-carbobenzoxy-L-leucinyl-L-leucinyl-L-leucinal (auch bezeichnet als MG 132 oder zLLL), dessen Borsäure-Derivat MG232; N-Carbobenzoxy-Leu-Leu-Nva-H (bezeichnet als MG115; N-Acetyl-L-Leuzinyl-L-Leuzinyl-L-Norleuzinal (bezeichnet als LLnL), N-Carbobenzoxy-Ile-Glu(OBut)-Ala-Leu-H (auch bezeichnet als PSI);
8.c) Peptide, welche C-terminal eine α,β-Epoxyketon-Struktur tragen, ferner Vinyl-sulfone, wie
8.c)1. Carbobenzoxy-L-Leucinyl-L-Leucinyl-L-Leucin-vinyl-sulfon oder
8.c)2. 4-Hydroxy-5-iodo-3-nitrophenylactetyl-L-Leucinyl-L-Leucinyl-L-Leucin-vinyl-sulfon (NLVS)
8.d) Glyoxal- oder Borsäure-Reste, wie
8.d)1. Pyrazyl-CONH(CHPhe)CONH(CHisobutyl)B(OH)₂) sowie
8.d)2. Dipeptidyl-Borsäure-Derivate oder
8.e) Pinacol-Ester - wie Benzyloxycarbonyl(Cbz)-Leu-Leu-boroLeu-Pinacol-Ester.

9. Proteasom-Inhibitoren nach Anspruch 8, **dadurch gekennzeichnet, dass** als besonders geeignete Proteasom-Inhibitoren die Epoxyketone
9.1. Epoxomicin (Epoxomycin, Molekülformel: C₂₈H₈₆N₄O₇) und/oder
9.2. Eponemicin (Eponemycin, Molekülformel: C₂₀H₃₆N₂O₅)
eingesetzt werden.

10. Proteasom-Inhibitoren nach Anspruch 8, **dadurch gekennzeichnet, dass** als besonders geeignete Proteasom-Inhibitoren aus der PS-Serie die Verbindungen
10.1. UPS-519 als β-Lacton - sowie als Lactacystin-Derivat die Verbindung 1R-[1S, 4R,5S]]-1-(1-Hydroxy-2-methylpropyl)-4-propyl-6-oxa-2-azabicyclo[3.2.0]heptane-3,7-dione - Molekülformel C₁₂H₁₉NO₄ - und/oder
10.2. PS-314 als Peptidyl-Borsäure-Derivat die Verbindung N-Pyrazinecarbonyl-L-Phenylalanin-L-Leuzin-Borsäure - Molekülformel C₁₉H₂₅BN₄O₄ - und/oder
10.3. PS-273 (Morpholin-CONH-(CH-Naphthyl)-CONH-(CH-isobutyl)-B(OH)2) und dessen Enantiomer PS-293 und/oder
10.4. die Verbindung PS-296 (8-Quinolyl-sulfonyl-CONH-(CH-Napthyl)-CONH(-CH-isobutyl)-B(OH)₂) und/oder
10.5. PS-303 (NH₂(CH-Naphthyl)-CONH-(CH-isobutyl)-B(OH)₂) und/oder
10.6. PS-321 als (Morpholin-CONH-(CH-Napthyl)-CONH-(CH-Phenylalanin)-B(OH)₂); - und/oder
10.7. PS-334 (CH₃-NH-(CH-Naphthyl-CONH-(CH-Isobutyl)-B(OH)₂) und/oder
10.8. die Verbindung PS-325 (2-Quinol-CONH-(CH-*homo*-Phenylalanin)-CONH-(CH-isobutyl)-B(OH)₂) und/oder
10.9. PS-352 (Phenyalanin-CH₂-CH₂-CONH-(CH-Phenylalanin)-CONH-(CH-isobutyl)1-B(OH)₂) und/oder
10.10. PS-383 (Pyridyl-CONH-(CH*p*F-Phenylalanin)-CONH-(CH-isobutyl)-B(OH)₂) eingesetzt werden.

11. Proteasom-Inhibitoren nach einem der Ansprüche 1 bis 10 zur Unterdrückung von Flavivirus-Infektionen und Pestivirus-Infektionen bei Menschen und Tieren.

12. Proteasom-Inhibitoren nach einem der Ansprüche 1 bis 10 zur
a) Regeneration von Patienten nach Flavivirus-Infektionen oder
b) Regeneration von Stalltieren nach Flavivirus- oder Pestivirus-Infektionen.

13. Proteasom-Inhibitoren nach einem der Ansprüche 1 bis 10 zur Hemmung sowohl der Erhaltung und Persistenz einer bereits etablierten Infektion als auch einer Sekundärinfektion und somit der Ausbreitung einer Infektion, einschließlich der Blockierung der Ausbreitung einer Flaviviridae-Infektion in vivo.

14. Proteasom-Inhibitoren nach einem der Ansprüche 1 bis 10 in Kombination untereinander zur Behandlung und Bekämpfung von Flavivirus-bedingtem Fieber, Hämorrhagien und Encephalitiden sowie Pestivirus-verursachten Krankheiten.

15. Proteasom-Inhibitoren nach einem der Ansprüche 1 bis 10 in Kombination mit bereits in der anti-viralen Therapie von Flaviviridae-Infektionen verwendeten Therapeutika.

16. Proteasom-Inhibitoren nach einem der Ansprüche 1 bis 10 zur Behandlung von Koinfektionen verschiedener Flaviviren und Pestiviren.

17. Proteasom-Inhibitoren nach einem der Ansprüche 1 bis 10 zur Verhinderung der Etablierung einer systemischen Flaviviridae-Infektion unmittelbar nach Kontakt mit infektiösem Virus.

18. Proteasom-Inhibitoren nach einem der Ansprüche 1 bis 10 zur Vorbeugung einer Flaviviridae-Infektion bei Personen mit hohem Risiko einer Neuinfektion, wie bei Ärzten, Risiko-Personal in Häusern mit hohem Besucherverkehr, Drogenabhängigen, Reisenden in hochendemische Gebiete für Flaviviridae, in der Krankenbehandlung oder für Familienangehörige von chronischen Virusträgern.

## Claims

1. Proteasome inhibitors for use in a method for treatment of diseases which are caused by the genera Flavivirus or Pestivirus of the family Flaviviridae.

2. Proteasome inhibitors according to claim 1 for treatment and prophylaxis of fever, hemorrhages, leukopenia, thrombocytopenia, diarrhetic diseases and encephalitides causally related to a Flavivirus, as well as diseases causally related to a Pestivirus.

3. Proteasome inhibitors according to claim 1 or 2, wherein substances are used
3.1. which inhibit, regulate or otherwise influence the activities of the ubiquitin/proteasome-pathway
3.2. which influence in particular the enzymatic activities of the entire 26S proteasome complex and
3.3. which influence in particular the enzymatic activities of the free 20S catalytically active proteasome complex not assembled with regulatory subunits.

4. Proteasome inhibitors according to any one of claims 1 to 3, wherein substances are used, which are absorbed as proteasome inhibitors by cells of higher eukaryotes and which, after cell absorption, interact with the catalytic subunits of the proteasome and thereby block all or individual proteolytic activities of the proteasome - the trypsin, chymotrypsin and postglutamyl-peptide hydrolyzing activities - within the 26S or also the 20S proteasome complex.

5. Proteasome inhibitors according to any one of claims 1 to 4, wherein they are used together with other substances influencing, regulating or inhibiting the cellular ubiquitin system such as the activities
5.1. of the ubiquitin conjugating enzymes and/or
5.2. of the ubiquitin hydrolyzing enzymes.

6. Proteasome inhibitors according to any one of claims 1 to 5, wherein substances are used, which can be administered in different forms *in vivo* orally, intravenously, intramuscularly, subcutaneously or in encapsulated form with or without modifications carrying cell specificity, present low cytotoxicity due to utilization of a specific application and/or dosage regime, cause no or insignificant side effects, have a relatively high metabolic half-life and a relatively low clearance rate in the organism.

7. Proteasome inhibitors according to any one of claims 1 to 6, wherein substances are used, which
a) are isolated in natural form from microorganisms or other natural sources or
b) result from natural substances by chemical modifications or
c) are produced by total synthesis or
d) are synthesized by gene therapeutic procedures *in vivo.*

8. Proteasome inhibitors according to claims 1 to 7, wherein substances are used which belong to the following substance classes:
8.a) naturally occurring proteasome inhibitors:
- peptide derivatives comprising C-terminal epoxyketone structures
- β-lactose derivatives
- aclacinomycin A (also called aclarubicin)
- lactacystin and its chemically modified variants, such as the cell membrane penetrating variant "clastolactacystein β-lactose"
8.b) synthetically produced proteasome inhibitors:
- modified peptide aldehydes such as N-carbobenzoxy-L-leucinyl-L-leucinyl-L-leucinal (also called MG132 or zLLL), its boric acid derivative MG232; N-Carbobenzoxy-Leu-Leu-Nva-H (called MG115; N-Acetyl-L-Leuzinyl-L-Leuzinyl-L-Norleuzinal (called LLnL), N-Carbobenzoxy-Ile-Glu(OBut)-Ala-Leu-H (also called PSI);
8.c) Peptides carrying a C-terminal α,β-epoxyketone structure, moreover, vinyl sulfones such as
8.c)1. Carbobenzoxy-L-Leucinyl-L-Leucinyl-L-Leucin-vinyl-sulfone or
8.c)2. 4-Hydroxy-5-iodo-3-nitrophenylactetyl-L-Leucinyl-L-Leucinyl-L-Leucin-vinyl-sulfone (NLVS)
8.d) Glyoxal or boric acid residues such as
8.d)1. Pyrazyl-CONH(CHPhe)CONH(CHisobutyl)B(OH)₂) as well as
8.d)2. Dipeptidyl-boric acid derivatives or
8.e) Pinacol-esters - such as Benzyloxycarbonyl(Cbz)-Leu-Leu-boroLeu-Pinacol-ester.

9. Proteasome inhibitors according to claim 8, wherein as particularly suitable proteasome inhibitors the epoxyketones
9.1. epoxomicin (epoxomycin, molecular formula: C₂₈H₈₆N₄O₇) and/or
9.2. eponemicin (eponemycin, molecular formula: C₂₀H₃₆N₂O₅)
are used.

10. Proteasome inhibitors according to claim 8, wherein as particularly suitable proteasome inhibitors from the PS series the following compounds are used
10.1. PS-519 as β-lactose - as well as a lactacystin derivative the compound 1R-[1S, 4R,5S]]-1-(1-Hydroxy-2-methylpropyl)-4-propyl-6-oxa-2-azabicyclo[3.2.0]heptane-3,7-dione - molecular formula C₁₂H₁₉NPO₄ - and/or
10.2. PS-314 as a peptidyl-boric acid derivative the compound N-Pyrazinecarbonyl-L-Phenylalanine-L-Leucine-boric acid - molecular formula C₁₉H₂₅BN₄O₄ - and/or 10.3. PS-273 (Morpholine-CONH-(CH-Naphthyl)-CONH-(CH-isobutyl)-B(OH)2) and its enantiomer PS-293 and/or
10.4. the compound PS-296 (8-Quinolyl-sulfonyl-CONH-(CH-Napthyl)-CONH(-CH-isobutyl)-B(OH)₂) and/or
10.5. PS-303 (NH₂(CH-Naphthyl)-CONH-(CH-isobutyl)-B(OH)₂) and/or
10.6. PS-321 as (Morpholine-CONH-(CH-Napthyl)-CONH-(CH-Phenylalanine)-B(OH)₂); - and/or
10.7. PS-334 (CH₃-NH-(CH-Naphthyl-CONH-(CH-Isobutyl)-B(OH)₂) and/or
10.8. the compound PS-325 (2-Quinol-CONH-(CH-*homo*-Phenylalanine)-CONH-(CH-isobutyl)-B(OH)₂) and/or
10.9. PS-352 (Phenyalanin-CH₂-CH₂-CONH-(CH-Phenylalanine)-CONH-(CH-isobutyl)1-B(OH)₂) and/or
10.10. PS-383 (Pyridyl-CONH-(CH*p*F-Phenylalanine)-CONH-(CH-isobutyl)-B(OH)₂).

11. Proteasome inhibitors according to any one of claims 1 to 10 for the suppression of Flavivirus infections and Pestivirus infections in humans and animals.

12. Proteasome inhibitors according to any one of claims 1 to 10 for
a) recovery of patients after Flavivirus infections or
b) recovery of stable-held animals after Flavivirus or Pestivirus infections.

13. Proteasome inhibitors according to any one of claims 1 to 10 for inhibition not only of the sustainment and persistence of an already established infection but also of a secondary infection and thus the spread of an infection, including blocking the spread of a Flaviviridae infection *in vivo*.

14. Proteasome inhibitors according to any one of claims 1 to 10 in combination among each other for treatment and fighting of fever, hemorrhages and encephalitides causally related to a Flavivirus, as well as diseases causally related to a Pestivirus.

15. Proteasome inhibitors according to any one of claims 1 to 10 in combination with therapeutics already used in antiviral therapy of Flaviviridae infections.

16. Proteasome inhibitors according to any one of claims 1 to 10 for treatment of co-infections of various Flaviviruses and Pestiviruses.

17. Proteasome inhibitors according to any one of claims 1 to 10 for preventing the establishment of a systemic Flaviviridae infection immediately after contact with an infectious virus.

18. Proteasome inhibitors according to any one of claims 1 to 10 for prevention of a Flaviviridae infection in persons having a high risk of infection such as physicians, risk personnel in places experiencing high visitor numbers, drug addicts, travellers to highly endemic regions for Flaviviridae, in treatment of sick persons or for family members of chronically virus- carrying individuals.

## Revendications

1. Inhibiteurs du Protéasome pour utilisation dans un procédé pour le traitement des maladies causées par les genres Flavivirus ou Pestivirus de la famille des Flavirididae.

2. Inhibiteurs du Protéasome selon la revendication 1 pour le traitement et la prophylaxie de la fièvre dû au Flavivirus, des haemorraghies, de la leucopénie, de la thrombocythopénie, des maladies de diarrhée et des encephalitides ainsi que des maladies causées par le Pestivirus.

3. Inhibiteurs du Protéasome selon la revendication 1 ou 2, **caractérisé en ce que** des substances sont utilisées
3.1. inhibant, réglant ou affectant autrement les activités de la voie ubiquitine-protéasome,
3.2. affectant notamment les activités enzymatiques du complexe entier protéasome 26S et
3.3. notamment les activités enzymatiques du complexe libre protéasome 20S catalytiquement actif non assemblé par des sous-ensembles régulateurs.

4. Inhibiteurs du Protéasome selon l'une quelconque des revendications 1 à 3, **caractérisé en ce que** des substances sont utilisées qui sont absorbées en tant qu'inhibiteurs du protéasome par des cellules des eucaryotes supérieurs et après absorption cellulaire interagissent avec les sous-ensembles catalytiques du protéasome en bloquant toutes les activités protéolytiques ou des activités protéolytiques individuelles du protéasome - les activités hydrolisant le trypsine-, le chymotrypsine- et le postglutamyle-peptide - endéans le complexe protéasome 26S ou aussi le complexe protéasome 20S.

5. Inhibiteurs du Protéasome selon l'une des revendications 1 à 4, **caractérisé en ce qu'**ils sont utilisés ensemble avec d'autres substances influençant, régulant ou inhibant le système cellulaire ubiquitine comme les activités
5.1. des enzymes conjuguant l'ubiquitine et/ou
5.2. des enzymes hydrolisant l'ubiquitine.

6. Inhibiteurs du Protéasome selon l'une des revendications 1 à 5, **caractérisé en ce que** des substances sont utilisées qui peuvent être administrées sous des formes différentes *in vivo* oralement, intraveineusement, intramusculairement, sous-cutanément ou sous forme capsulée avec ou sans des modifications portant une spécificité cellulaire, qui présentent une cytotoxicité faible en vertu de l'utilisation d'un certain régime d'application et/ou de dosage, qui ne suscitent pas des effets secondaires ou suscitent des effets secondaires insignifiants et qui ont une demi-vie métabolique relativement élevée et un taux de *clearance* relativement faible dans l'organisme.

7. Inhibiteurs du Protéasome selon l'une quelconque des revendications 1 à 6, **caractérisé en ce que** des substances sont utilisées qui
a) sont isolées sous forme naturelle à partir des microorganismes ou d'autres sources naturelles ou
b) proviennent des substances naturelles par modifications chimiques ou
c) sont produits entièrement de manière synthétique ou
d) sont synthétisés *in vivo* par des procédés de gène thérapeutique.

8. Inhibiteurs du Protéasome selon l'une quelconque des revendications 1 à 7, **caractérisé en ce que** des substances sont utilisées appartenant à des classes de substances suivantes:
8.a) des inhibiteurs du protéasome naturels:
- dérivés de peptide contenant des structures d'epoxyketone terminal C
- dérivés β-lactone
- Aclacinomycin A (aussi nommé Aclarubicin)
- Lactacystin et ses variantes modifiées chimiquement comme la variante "Clastolactacystéine β-Lactone" pénétrant la membrane cellulaire 8.b) des inhibiteurs du protéasome produits synthétiquement:
- des aldéhydes modifiés de peptide comme N-carbobenzoxy-L-leucinyle-L-leucinyld-L-leucinale (aussi nommé MG132 ou zLLL), son dérivé d'acide borique MG232; N-Carbobenzoxy-Leu-Leu-Nva-H (nommé MG115; N-Acetyle-L-Leuzinyle-L-Leuzinyle-L-Norleuzinale (nommé LLnL), N-Carbobenzoxy-Ile-Glu(OBut)-Ala-Leu-H (aussi nommé PSI);
8.c) des peptides portant en terminal C une structure α,β-epoxyketone, de plus, des vinyl-sulfones comme
8.c)1. Carbobenzoxy-L-Leucinyle-L-Leucinyle-L-Leucine-vinyl-sulfon ou
8.c)2. 4-Hydroxy-5-iodo-3-nitrophenylactetyle-L-Leucinyle-L-Leucinyle-L-Leucine-vinyl-sulfon (NLVS)
8.d) des résidus de glyoxal ou d'acide borique comme
8.d)1. Pyrazyle-CONH(CHPhe)CONH(CHisobutyle)B(OH)₂) ainsi que
8.d)2. des dérivés de dipeptidyle-acide borique ou
8.e) ester de pinacol - comme Benzyloxycarbonyle(Cbz)-Leu-Leu-boroLeu-Pinacol-Ester.

9. Inhibiteurs du Protéasome selon la revendication 8, **caractérisé en ce que** les epoxyketones
9.1 epoxomicin (epoxomycin, formule moléculaire: C₂₈H₈₆N₄O₇) et/ou
9.2 eponemicin (eponemycin, formule moléculaire: C₂₀H₃₆N₂O₅)
sont utilisés en tant que inhibiteurs du protéasome particulièrement appropriés.

10. Inhibiteurs du Protéasome selon la revendication 8, **caractérisé en ce que** sont utilisés en tant que inhibiteurs du protéasome particulièrement appropriés de la série PS les composés
10.1. PS-519 en tant que β-lactose - ainsi que en tant que dérivé de lactacystin le composé 1R-[1S,4R,5S]]-1-(1-Hydroxy-2-methylpropyle)-4-propyle-6-oxa-2-azabicyclo[3.2.0]heptane-3,7-dione - formule moléculaire C₁₂H₁₉NO₄ - et/ou
10.2. PS-314 en tant que dérivé de peptidyle- acide borique le composé N-Pyrazinecarbonyle-L-Phenylalanine-L-Leuzin-acide borique - formule moléculaire C₁₉H₂₅BN₄O₄ - et/ou
10.3. PS-273 (Morpholine-CONH-(CH-Naphthyle)-CONH-(CH-isobutyle)-B(OH)2) et son enantiomère PS-293 et/ou
10.4. le composé PS-296 (8-Quinolyle-sulfonyle-CONH-(CH-Napthyle)-CONH(-CH-isobutyle)-B(OH)₂) et/ou
10.5. PS-303 (NH₂(CH-Naphthyle)-CONH-(CH-isobutyle)-B(OH)₂) et/ou
10.6. PS-321 en tant que (Morpholine-CONH-(CH-Napthyle)-CONH-(CH-Phenylalanine)-B(OH)₂); - et/ou
10.7. PS-334 (CH₃-NH-(CH-Naphthyle-CONH-(CH-Isobutyle)-B(OH)₂) et/ou
10.8. le composé PS-325 (2-Quinol-CONH-(CH-homo-Phenylalanine)-CONH-(CH-isobutyle)-B(OH)₂) et/ou
10.9. PS-352(Phenyalanine-CH₂-CH₂-CONH-(CH-Phenylalanine)-CONH-(CH-isobutyle)1-B(OH)₂) et/ou
10.10. PS-383 (Pyridyle-CONH-(CHpF-Phenylalanine)-CONH-(CH-isobutyle)-B(OH)₂).

11. Inhibiteurs du Protéasome selon l'une quelconque des revendications 1 à 20 pour la suppression des infections à Flavivirus et Pestivirus auprès des hommes et des animaux.

12. Inhibiteurs du Protéasome selon l'une quelconque des revendications 1 à 10 pour
a) la régénération des patients après des infections à Flavivirus ou
b) la régénération des animaux de l'étable après des infections à Flavivirus ou à Pestivirus.

13. Inhibiteurs du Protéasome selon l'une quelconque des revendications 1 à 10 pour l'inhibition non seulement du maintien et de la persistance d'une infection déjà établie mais aussi d'une infection secondaire et donc de la propagation d'une infection, y compris le blocage d'une propagation d'une infection à Flaviviridae in vivo.

14. Inhibiteurs du Protéasome selon l'une quelconque des revendications 1 à 10 en combinaison entre eux pour le traitement et la lutte contre de la fièvre, des haemorraghies et des encephalitides dû au Flavivirus ainsi que contre des maladies causées par le Pestivirus.

15. Inhibiteurs du Protéasome selon l'une quelconque des revendications 1 à 10 en combinaison avec des médicaments utilisés déjà dans la thérapie antivirale des infections à Flaviviridae.

16. Inhibiteurs du Protéasome selon l'une quelconque des revendications 1 à 10 pour le traitement des co-infections des différents flavivirus et pestivirus.

17. Inhibiteurs du Protéasome selon l'une quelconque des revendications 1 à 10 pour prévenir l'établissement d'un infection systémique à Flaviviridae immédiatement après contact avec un virus infectieux.

18. Inhibiteurs du Protéasome selon l'une quelconque des revendications 1 à 10 pour la prévention d'une infection à Flaviviridae auprès des personnes avec un risque élevé d'une infection nouvelle comme pour les médecins, le personnel au risque dans des maisons avec un grand nombre de visiteurs, les toxicomanes, les voyageurs dans des régions hautement endémiques pour des Flaviviridae, dans le traitement des malades ou pour des membres de la famille des porteurs chroniques de virus.
